# EUROPEAN PATENT APPLICATION

(11) **EP 1 860 197 A1**
(43) Date of publication of application: **28.11.2007**
(21) Application number: 06010966.7
(22) Date of filing: 24.05.2006
(51) Int. Cl.: C12Q 1/68

(54) **Method for detecting target nucleic acids using template catalyzed transfer reactions**

(71) Applicant: Humboldt-Universität zu Berlin, D-10099 Berlin (DE)
(72) Inventor: Seitz, Oliver, 10407 Berlin (DE); Grossmann, Tom, 10245 Berlin (DE)
(74) Representative: Zwicker, Jörk

(57) **Abstract**

The present invention relates to the detection and quantification of nucleic acid sequences and to the sequence determination of nucleic acids using template catalyzed transfer reactions. The invention also relates to methods, reagents, and kits for detecting and quantifying nucleic acid sequences and for determining the sequence of nucleic acids.

## Description

The present invention relates to the detection and/or quantification of nucleic acid sequences and to the sequence determination of nucleic acids using template catalyzed transfer reactions. The invention also relates to methods, reagents, and kits for detecting nucleic acid sequences and for determining the sequence of nucleic acids.

### BACKGROUND OF THE INVENTION

Due to the importance of single nucleotide polymorphisms (SNPs) for the occurrence of a number of diseases and due to their influence on the effectiveness of medicaments, a number of diagnostic methods for their detection have been developed. The methods can be classified according to their approaches into heterogeneous and homogenous assays.

Heterogeneous assays are based on the immobilisation of either the probe or the analyte on a solid or gel phase, enabling the separation of unbound binding partners. Heterogeneous assays have the advantage that they may be employed in high throughput formats and can be well automated. They are well suited for mass screenings, but they are of limited use for the highly selective detection of a known mutation in clinical routine. Moreover, the need for washing steps prevents a real-time detection and the *in vivo* use.

In homogeneous assays, the analyte and the detection system exist simultaneously in the liquid phase. Therefore, it is necessary in an application that the detection of the analyte is linked to the change of a detectable value. Often fluorescence dyes are used, the spectral properties of which are modified by the detection system, thus allowing a direct observation.

Enzymatic procedures make use of proteins, which participate in replication, translation or repair of DNA. The high selectivity of the enzymes when carrying out the reactions is the basis for the following methods. A basis for many of these assays is the polymerase chain reaction (PCR). Drawbacks of enzymatic reactions are their low tolerance against substrate modifications, the low activity at RNA targets, the exclusion of *in vivo* application, and high cost.

Examples for such enzymatic methods are the allele specific amplification, primer extension assay, invader assay, TaqMan® assay, and the oligonucleotide ligation assay (OLA).

The application of inert probes for the detection of nucleic acid sequences makes use of the differences in stability between perfectly matched and single nucleotide mismatched duplexes. The commonly employed oligonucleotide probes have a length of 16 to 20 bases, because probes with this length and longer probes statistically cover a unique region of the genome. This approach allows a wide variety of probe modifications, so that nucleic acid analogues can also be employed. The probes described below can be used in combination with PCR, thus allowing real-time detection of DNA sequences. They also allow the *in vivo* detection of DNA and RNA sequences.

Examples of such inert probes are High Beacons^{™} (French, D.J. et al (2001) Mol. Cell. Probes 15, 363-374), Kissing/Hybridisation probes (Cardullo R.A. (1998) PNAS 85, 8790-8794), Light-up probes (Nielsen, P.E. (1991) Science 254, 1497-1500; Jenkins, Y. and Barton, J.K.(1992) J. Am. Chem. Soc. 114, 8736-8738; Ishiguro, T. et al. (1996) Nucleic Acids Res. 24, 4992-4997; Uhlmann, E. et al. (1998) Angew. Chemie.-Int. Edit., 37, 2797-2823), competitive hybridisation probes (Morrison, L.E. et al. (1989) Anal. Biochem. 183, 231-244) like Molecular Beacons (Tyagi, S. and Kramer, F.R. (1996) Nat. Biotechnol. 14, 303-308; Tyagi, S. et al. (1998) Nat. Biotechnol. 16, 49-53), MagiProbes (Yamane, A. (2002) Nucleic Acids Res. 30, e97), intercalating nucleic acids (INAs) (Christensen, U.B. and Pedersen E.B. (2003) Helv. Chim. Acta 86, 2090-2097), and forced intercalation probes (FIT probes) (Köhler, O. and Seitz, O. (2003) Chem. Commun. 2938-2939; Köhler, O. et al. (2005) Chembiochem 6, 69-77). Although the selectivity of inert probes towards single nucleotide mismatches could be improved by the development of Molecular Beacons and FIT probes, the selectivity of DNA detection by hybridization of inert probes does not reach that of enzymatic systems. One reason for this is the great length of the probes, which is necessary to guarantee the uniqueness of the section of the sequence.

The hybridisation of short oligonucleotides proceeds with higher selectivity compared to long oligonucleotides. Thus, to achieve a high sequence specificity the sequence part to be detected can be divided into two adjacent short probes. In a chemical method of detection, the probes are designed in a way, that permits only upon simultaneous hybridisation of both oligonucleotides a probe modifying event, which is subsequently detected. Thus, on the one hand, the uniqueness of the section of the sequence is guaranteed, and on the other a short and consequently selectively binding probe can be employed. With this approach, selectivities can be achieved which lie in the range of the above listed enzymatic methods. In chemical ligation reactions a broad variety of substrates and reactions can be employed, allowing also an *in vivo* detection of DNA and RNA sequences.

Most of the chemical methods for SNP detection employ the template mediated ligation of two modified oligonucleotides. Different types of reactions are used dependent on the type of probes employed. In the case of DNA probes, imine formation and phosphorothioate linkages have been used. For PNA probes the use of chemical ligation of thioesters with cysteine derivatives was reported.

The yield of the ligation is most often determined by gel electrophoresis or high performance liquid chromatography (HPLC). To use fluorescence based assays has the advantage of monitoring the progress of the reaction in real-time. In a system based on fluorescence resonance energy transfer (FRET), one of the probes was labelled with a fluorescence donor, the other one with a fluorescence acceptor. Due to the template mediated ligation of the two oligonucleotides, both fluorophores were positioned in direct vicinity, resulting in FRET and emission of the fluorescence acceptor. A further fluorescence based read out system has been realized with "QUAL probes" (Sando, S. and Kool, E.T. (2002) J. Am. Chem. Soc. 124, 9686-9687; Sando, S. and Kool, E.T. (2002) J. Am. Chem. Soc. 124, 2096-2097; Abe, H. and Kool, E.T. (2004) J. Am. Chem. Soc. 126, 13980-13986; Sando, S. et al. (2004) J. Am. Chem. Soc. 126, 1081-1087; Silverman A.P. and Kool, E.T. (2005) Trends Biotechnol. 23, 225-230; Silverman A.P. and Kool, E.T. (2005) Nucleic Acids Res. 33, 4978-4968). In these probes, a fluorophore and a fluorescence quencher are bound to one oligonucleotide. In the ligation reaction, the fluorescence quencher serves as a leaving group, whereby with proceeding of the reaction an increase of the fluorescence signal occurs. However, it is not possible to discern between the selective ligation reaction and the unselective background hydrolysis.

A general problem of the template mediated ligation reaction that prevents signal amplification is product inhibition. Product inhibition is the blockage of the template by the reaction product. In the case of ligation reactions, product inhibition is due to entropic reasons. The complex of the reactants is formed by three oligonucleotides, whereas the complex of the products is formed by two oligonucleotides, the ligation product and the analyte DNA. This leads to an increased stability of the product complex and, consequently, to a hindered replacement of the ligation product by the reactant probes. Therefore, a stoichiometric amount of analyte DNA is necessary for the quantitative conversion of reactants. This lowers the sensitivity of the assay making it necessary to amplify the analyte DNA by PCR prior to the detection of the mutation. It would be more practical and cost effective, if genomic DNA could be employed directly in a PCR free assay.

Different approaches have been taken to overcome product inhibition. For example, Lynn and co-workers (Goodwin, J.T. and Lynn, D.G. (1992) J. Am. Chem. Soc. 114, 9197-9198; Zhan, Z.Y.J. and Lynn, D.G. (1997) J. Am. Chem. Soc. 119, 12420-12421; Li, Z.Y. et al. (2002) J. Am. Chem. Soc. 124, 746-747) used very short probe lengths, which led to fast exchange kinetics of strands, but also to a reduction of the number of nucleotides in the probes, which are necessary for specific hybridization. It was also shown that the stability of the formed product template duplexes is reduced in reactions in which the ligation occurs opposite to an unpaired base. This leads at the same time to an increase of selectivity towards single base mismatches. A further possibility to lower the stability of the product complex is the use of flexible linkers at the ligation site. But also in these approaches, the stability of the product complex could not be lowered to that extent, that the ligation product is replaced in sufficient amounts by the reactants. Hence, only very low yields could be obtained with substoichiometric amounts of analyte DNA.

In the template mediated O-deacylation, an approach was realised for modified PNA probes, in which product complexes and reactant complexes exhibit a similar stability. One of the probes carries an ester function and the other one a functional group causing the deacylation of the alcohol function. After the reaction, two isolated probes are still present, which can easily be displaced from the template by the reactants. Hydrolysis of the ester by probes modified by an imidazole residue or by a copper complex was reported. A further possibility for DNA induced deacylation of the alcohol is the Staudinger reaction, which leads to formation of a peptide bond.

The determination of the yield in these systems can be done with the use of HPLC. In a further approach, weakly fluorescing acyl coumarin or acyl fluoresceine derivatives have been employed, and the deacylation of these derivatives led to an increase in fluorescence intensity. However, in these methods, it is not possible to differentiate between the selective template mediated reaction and the unselective background hydrolysis. It also is a severe limitation that the template mediated reactions do not proceed fast enough to allow high turnover rates.

It was an aim of the present invention to overcome these and other disadvantages in the prior art and to provide a method, which allows fast and specific detection of small amounts of target nucleic acids in a sample. In particular, the method of the present invention allows differentiating between selective template-mediated reactions and unselective background hydrolysis.

### SUMMARY OF THE INVENTION

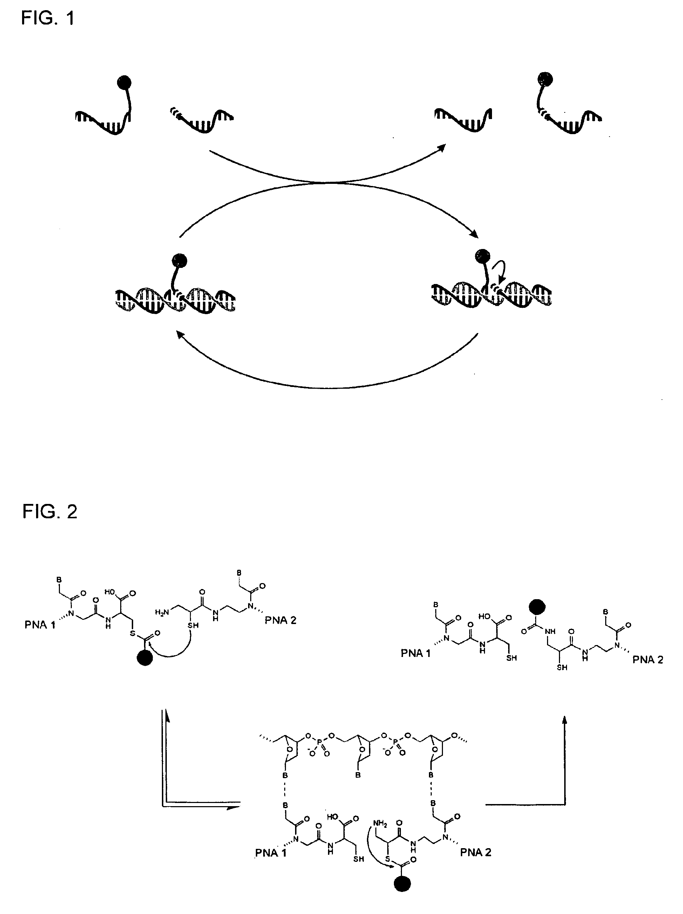

The inventors surprisingly found that the chemical ligation could be modified from a ligation reaction to a transfer reaction. The general principle of the transfer reaction of the present reaction is shown in Fig. 1. A preferred embodiment of the transfer reaction of the present invention is shown in Fig. 2.

The transfer reaction detailed in the present invention exhibits at least three advantages over the prior art: (i) the reaction is fast enough to allow a real-time detection of nucleic acids, (ii) the transfer of a reporter group allows to differentiate between the specific template-mediated reaction and unspecific background hydrolysis, and (iii) the reaction is not impaired by product inhibition, thus enabling the detection of substoichiometric amounts of target nucleic acids.

In a first aspect the present invention relates to a method for detecting at least one target nucleic acid sequence in a sample comprising the steps of:
(i) contacting the sample with at least one probe set for each target nucleic acid sequence, the probe set comprising:
   (a) a probe 1 comprising a first reporter group, which is capable of being transferred to a probe 2, and a region, which is complementary to a first region of the target nucleic acid sequence, and
   (b) a probe 2 comprising a region, which is complementary to a second region of the target nucleic acid sequence and a moiety which is capable of receiving said first reporter group when both probe 1 and probe 2 hybridize to the target nucleic acid,
   wherein said second region of the target nucleic acid sequence is adjacent to the first region of the target nucleic acid;
(ii) exposing the sample to conditions which lead to the transfer of the first reporter group to the probe 2; and
(iii) detecting probe 2 molecules to which said first reporter group has been transferred.

In a further aspect the invention relates to a kit for detecting at least one target nucleic acid sequence in a sample comprising one probe set for each target nucleic acid sequence, the probe set comprising:
(a) a probe 1 having the structure (VIII) 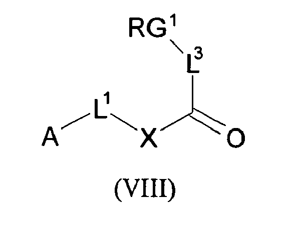 wherein
   - RG₁: is a first reporter group;
   - X: is S, O, Se, S-C(O), O-C(O), Se-C(O), or P⁺R¹R², wherein the C(O) group, if present, is bound to L¹;
   - R¹ and R²,: if present, are independently selected from the group consisting of aryl and alkyl;
   - L¹: is a linker or a bond; L³ is a linker or a bond;
   - A: comprises a region, which is complementary to a first region of the target nucleic acid sequence, and optionally a second reporter group which is linked to said region via a covalent bond or a linker;
   and
   (b) a probe 2 having the structure (IX) or (X) 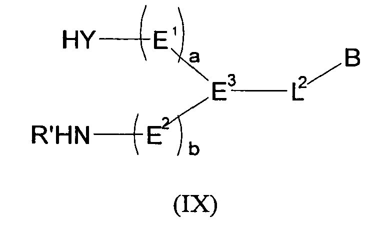 wherein
   E¹ and E² are independent of each other CHR", R" being a hydrogen, alkyl, alkenyl, alkynyl, cycloalkyl, heterocycloalkyl, aryl, heteroaryl, aralkyl, or a heteroaralkyl group, optionally substituted;
   E³ is selected from the group consisting of alkyl, alkenyl, heteroalkyl, and heteroalkenyl, cycloalkyl, heterocycloalkyl, alicyclic system, aryl or heteroaryl group; optionally substituted; and wherein E¹ and E² are attached to the same or to adjacent carbon and/or nitrogen atom(s); optionally substituted;
   R' is hydrogen, an alkyl, alkenyl, alkynyl, cycloalkyl, heterocycloalkyl, aryl, heteroaryl, aralkyl, or a heteroaralkyl group;
   Y is S or Se;
   L² is a linker or a bond;
   B comprises a region, which is complementary to a second region of the target nucleic acid sequence, and optionally a first reporter group which is linked to said region via a covalent bond or a linker; and one of a and b is 1 and the other one is 0 or both a and b are 1 or one of a and b is 2 and the other one is 0; 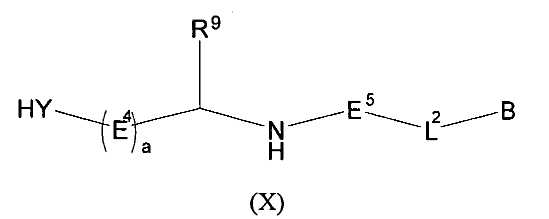 wherein
   E⁴ in each instance is independently CHR", wherein R" is hydrogen, an alkyl, alkenyl, alkynyl, cycloalkyl, heterocycloalkyl, aryl, heteroaryl, aralkyl, or a heteroaralkyl group, optionally substituted;
   E⁵ is CHR"' or CR"', wherein R"' is hydrogen, an alkyl, alkenyl, alkynyl, cycloalkyl, heterocycloalkyl, aryl, heteroaryl, aralkyl, or a heteroaralkyl group, optionally substituted;
   R⁹ is hydrogen; alkyl; alkenyl; alkynyl; cycloalkyl; heterocycloalkyl; aryl; heteroaryl; aralkyl; or a heteroaralkyl group; optionally substituted
   or R⁹ and R'" are taken together to form a heterocycloalkyl, alicylic system, or heteroaryl; optionally substituted;
   Y is S or Se;
   L² is a linker or a bond;
   B comprises a region, which is complementary to a second region of the target nucleic acid sequence, and optionally a first reporter group which is linked to said region via a covalent bond or a linker; and
   a is 1 or 2.
wherein the second region of the target nucleic acid sequence is adjacent to the first region of the target nucleic acid.

In a further aspect the present invention is directed to the use of the methods and kits of the present invention for determining the sequence of a target nucleic acid, for the detection of at least one single nucleotide polymorphism in at least one target nucleic acid, and for the detection of at least one target nucleic acid from at least one pathogenic or allergenic organism.

### DETAILED DESCRIPTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

The present invention provides methods, kits and uses for detecting the presence or absence of target nucleic acid sequences in a sample. By "nucleic acid" or "oligonucleotide" or grammatical equivalents thereof is meant at least two nucleotides covalently linked together. A nucleic acid of the present invention will generally contain phosphodiester bonds, although in some cases, as outlined below, particularly for use with probes, nucleic acid analogs are included that may have alternate backbones, comprising, for example, phosphoramide, phosphorothioate, phosphorodithioate, O-methylphosphoroamidite linkages, and peptide nucleic acid backbones and linkages. Other analog nucleic acids include those with positive backbones; non-ionic backbones and non-ribose backbones. Nucleic acids containing one or more carbocyclic sugars are also included within the definition of nucleic acids. These modification of the ribose-phosphate backbone may be done to facilitate the addition of labels, or to increase the stability and half-life of such molecules in physiological environments. As will be appreciated by those in the art, all of these nucleic acid analogs may find use in the present invention. In addition, mixtures of naturally occurring nucleic acids, such as DNA and RNA, and analogs can be made. Alternatively, mixtures of different nucleic acid analogs, and mixtures of naturally occurring nucleic acids and analogs can be made.

A "target" or "target sequence" according to the present invention comprises a specific nucleic acid sequence, the presence or absence of which is to be detected or the amount of which is to be quantified. The person of ordinary skill will appreciate that while the target sequence is generally described as a single-stranded molecule, the opposing strand of a double-stranded molecule comprises a complementary sequence and/or the double-strand that may also be used as a target. In certain embodiments, a target sequence comprises an upstream or 5' region, a downstream or 3' region, and a "pivotal nucleotide" located between the upstream region and the downstream region or located within the upstream region or located within the downstream region. In certain embodiments, the pivotal nucleotide is the nucleotide being detected by the probe set and may be represent, for example without limitation, a single polymorphic nucleotide in a multiallelic target locus.

As used herein, the term "sample" refers to any substance containing or presumed to contain a nucleic acid of interest (a target nucleic acid sequence) or which is itself a nucleic acid containing or presumed to contain a target nucleic acid sequence of interest. The term "sample" thus includes a sample of nucleic acid (genomic DNA, cDNA, RNA), cell, organism, tissue, fluid, or substance including but not limited to, for example, plasma, serum, spinal fluid, lymph fluid, synovial fluid, urine, tears, stool, external secretions of the skin, respiratory, intestinal and genitourinary tracts, saliva, blood cells, tumors, organs, tissue, samples of *in vitro* cell culture constituents, natural isolates (such as drinking water, seawater, solid materials), microbial specimens, food, drinks, and objects or specimens that have been marked with nucleic acid tracer molecules.

A "blank sample", as used herein, is any liquid or solid composition which does not contain the nucleic acid of interest. Preferably, a "blank sample" resembles the corresponding "sample" or "samples" in as many chemical and physical properties as possible (such as pH, ionic strength, viscosity; colour, spectral properties, concentration of salts, proteins, nucleic acids etc.). In certain embodiments, a "blank sample" may comprise water or a buffered solution.

A "probe" of the present invention comprises a region which is complementary to a region of a target nucleic acid sequence. In the following said "region which is complementary to a region of a target nucleic acid sequence" will be termed "complementary region" and said "region of a target nucleic acid sequence" will be termed "target region". Under appropriate hybridization conditions a "complementary region" of a probe of the present invention can anneal to the corresponding "target region". Typically, the annealing occurs via Watson-Crick base pairs, but annealing via reverse Watson-Crick base pairs, via Hoogsteen base pairs, reverse Hoogsteen base pairs, via Wobble base pairs and/or of minor groove binding hairpin polyamides (Poulin-Kerstien, A.T. and Dervan, P.B. (2003) J. Am. Chem. Soc. 125, 15811-15821) is also considered within the scope of the present invention. It is well known in the art that such annealing, especially in the case of Watson-Crick base pairs, is dependent in a rather predictable manner on several parameters, including temperature, ionic strength, probe length, and G:C content of the probes. The complementary regions of probe and target will preferably anneal under stringent conditions as defined in the art, preferably they will only anneal if there are less than 1, 2, 3, 4, S, or 6 mismatches between the analyt and the probe strand.

The "complementary region" typically is an oligonucleotide comprised of naturally occurring nucleic acids or of analogs of nucleic acids, or a hairpin polyamide as detailed above or of mixtures of naturally occurring nucleic acids and analogs of nucleic acids. Thus, the complementary region can consist of DNA, RNA, peptide nucleic acid (PNA), phosphorothioate DNA (PS-DNA), 2'-O-methyl RNA (OMe-RNA), 2'-O-methoxy-ethyl RNA (MOE-RNA), N3'-P5' phosphoroamidate (NP), 2'-fluoro-arabino nucleic acid (FANA), locked nucleic acid (LNA), morpholino phosphoroamidate (MF), cyclohexene nucleic acid (CeNA), or tricycle-DNA (tcDNA) or of mixtures of any of these naturally occurring nucleic acids and nucleic acid analogs (for a review see Kurreck J. "Antisense technologies", Eur. J. Biochem. 270, pp. 1628-1644 (2003)). The "complementary region" according to the invention typically has a length of 3 to 50 nucleotides, preferably from 5 to 35 nucleotides, more preferably from 6 to 25 nucleotides and most preferably from 6 to 15 nucleotides.

A probe of the present invention may comprise one or more reporter groups. These one or more reporter groups can be directly linked via a covalent bond to the "complementary region". In certain embodiments of the present invention each of the one or more reporter groups can be linked via a linker to the "complementary region". Any probe of the present invention can be linked to a stationary phase. This link to the stationary phase can be direct via a chemical bond or the probe may be linked via a linker. In embodiments, wherein more than one linker is present in a probe, the linkers can be identical or different.

A "probe set" according to the present invention comprises one first probe (probe 1) and one second probe (probe 2) that are capable to hybridize to adjacent regions of the same target nucleic acid sequence. The probe set can additionally comprise a third probe (probe 3), which is capable to hybridize to the same target nucleic acid sequence in a region which is adjacent to the region to which probe 1 hybridizes and/or to the region to which probe 2 hybridizes. The probe set may comprise one or more further probes, which are capable to hybridize to the same target nucleic acid sequence as the first probe, the second probe, and the third probe. The terms "first probe" and "probe 1" are used interchangeably throughout the present invention. Likewise, the terms "second probe" and "probe 2" as well as the terms "third probe" and "probe 3" are used interchangeably throughout the present invention.

The numbering of the probes, i.e. probe 1, probe 2, probe 3, etc., shall not be construed as an indication of the 5' (upstream) or 3' (downstream) orientation of the probes when hybridized to the target nucleic acid sequence. Thus, in certain embodiments of the present invention, probe 1 is designed to hybridize to the upstream region of the target nucleic acid sequence and probe 2 is designed to hybridize to the downstream region of the target nucleic acid sequence. In other embodiments of the present invention probe 2 is designed to hybridize to the upstream region of the target nucleic acid sequence and probe 1 is designed to hybridize to the downstream region of the target nucleic acid sequence. Likewise, probe 3 can be designed to hybridize upstream of probe 1 and/or probe 2; or probe 3 can be designed to hybridize downstream of probe 1 and/or probe 2.

"Probe 1" is defined as a probe comprising one or more reporter groups, wherein at least one of the reporter groups can be transferred to probe 2. When a probe 3 is present in the probe set, probe 1 may comprise one or more further reporter groups which may be transferred to probe 3. Probe 1 may comprise reporter groups which cannot be transferred to probe 2 or to probe 3, wherein these non-transferable reporter groups may or may not interact with the transferable reporter groups.

"Probe 2" is defined as a probe, which is capable of receiving at least one reporter group from probe 1. Probe 2 may comprise further reporter groups which may or may not interact with the at least one reporter group received from probe 1.

"Probe 3" is a probe which is either capable to transfer one or more reporter groups to probe 2 or it is capable to receive one or more reporter groups from probe 1. Probe 3 may comprise non-transferable reporter groups which may or may not interact with the transferable reporter groups.

When two or more probe sets are used in the methods, kits and uses of the present invention, the two or more probe sets may comprise different first probes or they may comprise the same first probe. Likewise, the two or more probe sets may comprise different second probes or they may comprise the same second probe. The two or more probe sets may also comprise any possible combination of first probes and second probes; i.e. the same first probe and the same second probe; the same first probe and different second probes; different first probes and the same second probe; or different first probes and different second probes. When the two or more probe sets comprise a third probe, the third probe may be the same in the two or more probe sets or different third probes may be used. This same third probe or these different third probes can be combined with any of the above detailed combinations of first probes and second probes.

The term "reporter group" as used herein refers to any tag, label or identifiable moiety. The person skilled in the art will appreciate that many reporter groups may be used in the present invention. For example, reporter groups include, but are not limited to, fluorophores, radioisotopes, chromogens, enzymes, antigens, heavy metals, dyes, magnetic probes, phosphorescence groups, chemiluminescent groups, and electrochemical detection moieties. Reporter groups also include elements of multi-element direct or indirect reporter systems, e.g. fluorophor/fluorescence quencher, fluorescence donor/fluorescence acceptor (i.e. FRET pair), biotin/(strept)avidin, antibody/antigen, ligand/receptor, enzyme/substrate, and the like, in which the element interacts which other elements of the system in order to effect a detectable (preferably quantifiable) signal. Detailed protocols for methods of attaching reporter groups to oligonucleotides and polynucleotides can be found in, among other places, G.T. Hermanson, Bioconjugate Techniques, Academic Press, San Diego, CA (1996) and S.L. Beaucage et al., Current Protocols in Nucleic Acid Chemistry, John Wiley & Sons, New York, NY (2000).

As used herein, "a reporter group which can be transferred" is a reporter group which is linked to a first molecule, preferably a first probe, in such a way that a chemical reaction with a second molecule, preferably a second probe, can take place, wherein after said chemical reaction the reporter group is linked to the second molecule. A probe "capable of receiving" a reporter group, as used herein, is a probe which comprises a moiety to which a reporter group can be transferred from another molecule, preferably another probe, in said chemical reaction, so that after said chemical reaction the reporter group is linked to said probe capable of receiving a reporter group. Conversely, it is preferred that the reporter groups which are not meant to be transferred are attached to the respective probe through bonds which are not prone to cleavage, e.g. hydrolysis, under the conditions under which "the reporter group, which can be transferred" is transferred. Accordingly, any change in signal can be attributed to the transfer and not additionally to the cleavage of the bond of any other reporter group.

As used herein, two nucleic acid sequences are termed "complementary" to each other, when only a section of the first nucleic acid sequence exhibits 100% complementarity to a section of the second nucleic acid sequence. Said section of the first nucleic acid sequence and said section of the second nucleic acid sequence preferably consist of 5 or more contiguous nucleotides, 10 or more contiguous nucleotides, 15 or more contiguous nucleotides, 20 or more contiguous nucleotides, or 25 or more contiguous nucleotides.

The term "adjacent to" is to be understood in that the distance between two regions of a nucleic acid sequence (i.e. two target regions) ranges in certain preferred embodiments from 0 to 10 nucleotides, preferably from 0 to 7 nucleotides, more preferably from 0 to 5 nucleotides, even more preferably from 0 to 3 nucleotides, and in the most preferred embodiments the distance is 1 or 2 nucleotides. The optimal distance between two probes to allow transfer of a reporter group will also depend on the length of the linker connecting the first reporter group to the part of the first probe hybridizing to the target nucleic acid and similarly also on the length of the linker linking the group to which the reporter will be transferred and the part of the second (or third) probe to which the reporter will be transferred. What is required for an efficient transfer is that both groups can make contact with each other in a way that allows the transfer to occur. Thus, in embodiments wherein the first reporter group is attached through a long linker it is possible to increase the distance between the two regions over the preferred range of 10 nucleotides and in embodiments in which only a short or no linker is provided distances of 0 to 4 nucleotides are preferred.

A "linker" is a moiety which links two different parts of a molecule. Typically a linker comprises an alkyl, preferably C₁ to C₅₀ alkyl, alkenyl, alkynyl, cycloalkyl, heterocycloalkyl, aryl, heteroaryl, aralkyl, or a heteroaralkyl moiety. It is preferred that the linker does not comprise any chemical groups which are capable of accepting the first reporter group, since this could lead to undesirable intramolecular reactions over the desired intermolecular reactions. In preferred embodiments, a linker is a peptide chain comprising naturally occurring amino acids and/or amino acid analogs. In a preferred embodiment of such a peptide linker the peptide would not have any free amino groups.

A "C nucleophile" is a moiety that comprises a nucleophilic carbon atom. Examples for such C-nucleophiles are Grignard reagents, cyanoalkyl, 4-nitrophenylalkyl, especially: nitroalkyl, enolates, 1,3-dicarbonyl compounds.

A small molecule is a compound with a preferable molecular weight below 1000 daltons.

In the following definitions of the terms: alkyl, heteroalkyl, cycloalkyl, heterocycloalkyl, aryl, aralkyl, heteroaryl, heteroaralkyl, alicyclic system, alkenyl, cycloalkenyl, and alkynyl are provided. These terms will in each instance of its use in the remainder of the specification have the respectively defined meaning and preferred meanings. Nevertheless in some instances of their use throughout the specification preferred meanings of these terms are indicated.

The term "alkyl" refers to a saturated straight or branched carbon chain. Preferably, the chain comprises from 1 to 10 carbon atoms, i.e. 1, 2, 3, 4, 5, 6, 7, 8, 9 or 10 e.g. methyl, ethyl, propyl, *iso*-propyl, butyl, *iso*-butyl, *tert*-butyl, pentyl, hexyl, pentyl, octyl. Alkyl groups are optionally substituted.

The term "heteroalkyl" refers to a saturated straight or branched carbon chain. Preferably, the chain comprises from 1 to 9 carbon atoms, i.e. 1, 2, 3, 4, 5, 6, 7, 8, 9 e.g. methyl, ethyl, propyl, *iso*-propyl, butyl, *iso*-butyl, sec-butyl, *tert*-butyl, pentyl, hexyl, pentyl, octyl, which is interrupted one or more times, e.g. 1, 2, 3, 4, 5, with the same or different heteroatoms. Preferably the heteroatoms are selected from O, S, and N, e.g. CH₂-O-CH₃, CH₂-O-C₂H₅, C₂H₄-O-CH₃, C₂H₄-O-C₂H₅ etc. Heteroalkyl groups are optionally substituted.

The terms "cycloalkyl" and "heterocycloalkyl", by themselves or in combination with other terms, represent, unless otherwise stated, cyclic versions of "alkyl" and "heteroalkyl", respectively, with preferably 3, 4, 5, 6, 7, 8, 9 or 10 atoms forming a ring, e.g. cyclopropyl, cyclobutyl, cyclopentyl, cyclohexyl, cycloheptyl, cyclooctyl etc. The terms "cycloalkyl" and "heterocycloalkyl" are also meant to include bicyclic, tricyclic and polycyclic versions thereof. If bicyclic, tricyclic or polycyclic rings are formed it is preferred that the respective rings are connected to each other at two adjacent carbon atoms, however, alternatively the two rings are connected via the same carbon atom, i.e. they form a spiro ring system or they form "bridged" ring systems. The term "heterocycloalkyl" preferably refers to a saturated ring having five of which at least one member is a N, O or S atom and which optionally contains one additional O or one additional N; a saturated ring having six members of which at least one member is a N, O or S atom and which optionally contains one additional O or one additional N or two additional N atoms; or a saturated bicyclic ring having nine or ten members of which at least one member is a N, O or S atom and which optionally contains one, two or three additional N atoms. "Cycloalkyl" and "heterocycloalkyl" groups are optionally substituted. Additionally, for heterocycloalkyl, a heteroatom can occupy the position at which the heterocycle is attached to the remainder of the molecule. Examples of cycloalkyl include cyclopropyl, cyclobutyl, cyclopentyl, cyclohexyl, 1-cyclohexenyl, 3-cyclohexenyl, cycloheptyl, spiro[3,3]heptyl, spiro[3,4]octyl, spiro[4,3]octyl, spiro[3,5]nonyl, spiro[5,3]nonyl, spiro[3,6]decyl, spiro[6,3]decyl, spiro[4,5]decyl, spiro[5,4]decyl, bicyclo[2.2.1]heptyl, bicyclo[2.2.2]octyl, adamantyl, and the like. Examples of heterocycloalkyl include 1-(1,2,5,6-tetrahydropyridyl), 1-piperidinyl, 2-piperidinyl, 3-piperidinyl, 4-morpholinyl, 3-morpholinyl, 1,8 diazo-spiro-[4,5] decyl, 1,7 diazo-spiro-[4,5] decyl, 1,6 diazo-spiro-[4,5] decyl, 2,8 diazo-spiro[4,5] decyl, 2,7 diazo-spiro[4,5] decyl, 2,6 diazo-spiro[4,5] decyl, 1,8 diazo-spiro-[5,4] decyl, 1,7 diazo-spiro-[5,4] decyl, 2,8 diazo-spiro-[5,4] decyl, 2,7 diazo-spiro[5,4] decyl, 3,8 diazo-spiro[5,4] decyl, 3,7 diazo-spiro[5,4] decyl, I-azo-7,11-dioxo-spiro[5,5] undecyl, 1,4-diazabicyclo[2.2.2]oct-2-yl, tetrahydrofuran-2-yl, tetrahydrofuran-3-yl, tetrahydrothien-2-yl, tetrahydrothien-3-yl, 1-piperazinyl, 2-piperazinyl, and the like.

The term "alicyclic system" refers to mono, bicyclic, tricyclic or polycyclic version of a cycloalkyl or heterocycloalkyl comprising at least one double and/or triple bond. However, an alicyclic system is not aromatic or heteroaromatic, i.e. does not have a system of conjugated double bonds/free electron pairs. Thus, the number of double and/or triple bonds maximally allowed in an alicyclic system is determined by the number of ring atoms, e.g. in a ring system with up to 5 ring atoms an alicyclic system comprises up to one double bond, in a ring system with 6 ring atoms the alicyclic system comprises up to two double bonds. Thus, the "cycloalkenyl" as defined below is a preferred embodiment of an alicyclic ring system. Alicyclic systems are optionally substituted.

The term "aryl" preferably refers to an aromatic monocyclic ring containing 6 carbon atoms, an aromatic bicyclic ring system containing 10 carbon atoms or an aromatic tricyclic ring system containing 14 carbon atoms. Examples are phenyl, naphthyl or anthracenyl. The aryl group is optionally substituted.

The term "aralkyl" refers to an alkyl moiety, which is substituted by aryl, wherein alkyl and aryl have the meaning as outlined above. An example is the benzyl radical. Preferably, in this context the alkyl chain comprises from 1 to 8 carbon atoms, i.e. 1, 2, 3, 4, 5, 6, 7, or 8, e.g. methyl, ethyl, propyl, *iso*-propyl, butyl, *iso*-butyl, *sec*-butenyl, *tert*-butyl, pentyl, hexyl, pentyl, octyl. The aralkyl group is optionally substituted at the alkyl and/or aryl part of the group. Preferably the aryl attached to the alkyl has the meaning phenyl, naphthyl or anthracenyl.

The term "heteroaryl" preferably refers to a five or six-membered aromatic monocyclic ring wherein at least one of the carbon atoms are replaced by 1, 2, 3, or 4 (for the five membered ring) or 1, 2, 3, 4, or 5 (for the six membered ring) of the same or different heteroatoms, preferably selected from O, N and S; an aromatic bicyclic ring system wherein 1, 2, 3, 4, 5, or 6 carbon atoms of the 8, 9, 10, 11 or 12 carbon atoms have been replaced with the same or different heteroatoms, preferably selected from O, N and S; or an aromatic tricyclic ring system wherein 1, 2, 3, 4, 5, or 6 carbon atoms of the 13, 14, 15, or 16 carbon atoms have been replaced with the same or different heteroatoms, preferably selected from O, N and S. Examples are furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl.

The term "heteroaralkyl" refers to an alkyl moiety, which is substituted by heteroaryl, wherein alkyl and heteroaryl have the meaning as outlined above. An example is the (2-pyridinyl) ethyl, (3-pyridinyl) ethyl, or (2-pyridinyl) methyl. Preferably, in this context the alkyl chain comprises from 1 to 8 carbon atoms, i.e. 1, 2, 3, 4, 5, 6, 7, or 8, e.g. methyl, ethyl methyl, ethyl, propyl, *iso*-propyl, butyl, *iso*-butyl, *sec*-butenyl, *tert*-butyl, pentyl, hexyl, pentyl, octyl. The heteroaralkyl group is optionally substituted at the alkyl and/or heteroaryl part of the group. Preferably the heteroaryl attached to the alkyl has the meaning oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, 2,3-benzodoazinyl, quinolinyl, isoquinolinyl, quinoxalinyl, quinazolinyl, quinolinyl, 1,2,3-benzotriazinyl, or 1,2,4-benzotriazinyl.

The terms "alkenyl" and "cycloalkenyl" refer to olefinic unsaturated carbon atoms containing chains or rings with one or more double bonds. Examples are propenyl and cyclohexenyl. Preferably, the alkenyl chain comprises from 2 to 8 carbon atoms, i.e. 2, 3, 4, 5, 6, 7, or 8, e.g. ethenyl, 1-propenyl, 2-propenyl, *iso*-propenyl, 1-butenyl, 2-butenyl, 3-butenyl, iso-butenyl, *sec*-butenyl, 1-pentenyl, 2-pentenyl, 3-pentenyl, 4-pentenyl, hexenyl, pentenyl, octenyl. Preferably the cycloalkenyl ring comprises from 3 to 8 carbon atoms, i.e. 3, 4, 5, 6, 7, or 8, e.g. 1-cyclopropenyl, 2-cyclopropenyl, 1-cyclobutenyl, 2-cyclobutenyl, 1-cyclopentenyl, 2-cyclopentenyl, 3-cyclopentenyl, cyclohexenyl, cyclopentenyl, cyclooctenyl.

The term "alkynyl" refers to unsaturated carbon atoms containing chains or rings with one or more triple bonds. An example is the propargyl radical. Preferably, the alkynyl chain comprises from 2 to 8 carbon atoms, i.e. 2, 3, 4, 5, 6, 7, or 8, e.g. ethynyl, 1-propynyl, 2-propynyl, 1-butynyl, 2-butynyl, 3-butynyl, 1-pentynyl, 2-pentynyl, 3-pentynyl, 4-pentynyl, hexynyl, pentynyl, octynyl.

In one embodiment, carbon atoms or hydrogen atoms in alkyl, cycloalkyl, aryl, aralkyl, alkenyl, cycloalkenyl, alkynyl radicals may be substituted independently from each other with one or more elements selected from the group consisting of O, S, N or with groups containing one ore more elements selected from the group consisting of O, S, N.

Embodiments include alkoxy, cycloalkoxy, aryloxy, aralkoxy, alkenyloxy, cycloalkenyloxy, alkynyloxy, alkylthio, cycloalkylthio, arylthio, aralkylthio, alkenylthio, cycloalkenylthio, alkynylthio, alkylamino, cycloalkylamino, arylamino, aralkylamino, alkenylamino, cycloalkenylamino, alkynylamino radicals.

Other embodiments include hydroxyalkyl, hydroxycycloalkyl, hydroxyaryl, hydroxyaralkyl, hydroxyalkenyl, hydroxycycloalkenyl, hydroxyalinyl, mercaptoalkyl, mercaptocycloalkyl, mercaptoaryl, mercaptoaralkyl, mercaptoalkenyl, mercaptocycloalkenyl, mercaptoalkynyl, aminoalkyl, aminocycloalkyl, aminoaryl, aminoaralkyl, aminoalkenyl, aminocycloalkenyl, aminoalkynyl radicals.

In another embodiment, hydrogen atoms in alkyl, cycloalkyl, aryl, aralkyl, alkenyl, cycloalkenyl, alkynyl radicals may be substituted independently from each other with one ore more halogen atoms. One radical is the trifluoromethyl radical.

If two or more radicals can be selected independently from each other, then the term "independently" means that the radicals may be the same or may be different.

### General

The present invention provides methods and kits for the detection and quantification of nucleic acid sequences and for the sequence determination of nucleic acids. The methods and kits useful in the invention typically employ template catalyzed transfer reactions of one or more reporter groups. The invention further provides uses of the methods and kits of the invention for the determination of a target nucleic acid sequence, for the detection of single nucleotide polymorphisms, and for the detection of pathogenic organisms.

### Embodiments of the Invention

The present invention provides a method for detecting at least one target nucleic acid sequence in a sample comprising the steps of:
(i) contacting the sample with at least one probe set for each target nucleic acid sequence,
   the probe set comprising:
   (a) a probe 1 comprising a first reporter group, which is capable of being transferred to a probe 2, and a region, which is complementary to a first region of the target nucleic acid sequence, and
   (b) a probe 2 comprising a region, which is complementary to a second region of the target nucleic acid sequence and a moiety which is capable of receiving said first reporter group when both probe 1 and probe 2 hybridize to the target nucleic acid,
      wherein said second region of the target nucleic acid sequence is adjacent to the first region of the target nucleic acid;
(ii) exposing the sample to conditions which lead to the transfer of the first reporter group to the probe 2; and
(iii) detecting probe 2 molecules to which said first reporter group has been transferred.

In a preferred embodiment of the invention the region of the probe 1 complementary to a first region of the target nucleic acid is selected from the group consisting of DNA, RNA, PNA, PS-DNA, OMe-RNA, MOE-RNA, NP, FANA, LNA, MF, CeNA and tcDNA.

In a further preferred embodiment of the invention the probe 1 comprises a second reporter group.

In a preferred embodiment of the invention the region of the probe 2 complementary to a second region of the target nucleic acid is selected from the group consisting of DNA, RNA, PNA, PS-DNA, OMe-RNA, MOE-RNA, NP, FANA, LNA, MF, CeNA and tcDNA.

In a further preferred embodiment of the invention the probe 2 comprises a first reporter group.

In a preferred embodiment of the probe set of the invention, the probe set further comprises a probe 3, which comprises a region which is complementary to a third region of the target nucleic acid, the probe 3 optionally comprising a first reporter group, wherein said third region is adjacent to the first region of the target nucleic acid or to the second region of the target nucleic acid.

In a preferred embodiment of the invention one or more reporter groups are selected from the group consisting of a fluorescent moiety, a quenching moiety, a donor fluorescent moiety, an acceptor fluorescent moiety capable to fluoresce upon transfer of energy from a donor fluorescent moiety, a radioactive moiety, a binding moiety. In a particularly preferred embodiment of the invention the one or more reporter groups are chosen in such that the transfer of a first reporter group of the probe 1 and/or the transfer of a second reporter group of the probe 1 allows detection of the probe 2 and/or probe 3. In a further preferred embodiment of the invention the one or more reporter groups are chosen in such that the transfer of a first reporter group of the probe 1 to probe 2 and/or the transfer of a first reporter group of the probe 3 to probe 2 allows detection of the probe 2 and/or probe 3.

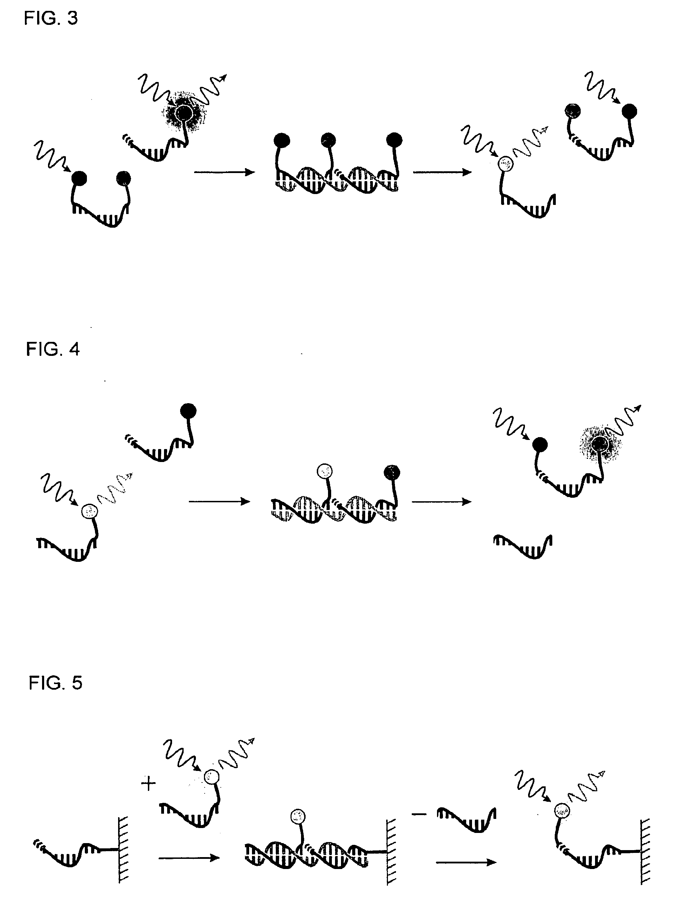

In particularly preferred embodiments of the invention
(a) the first reporter group of probe 1 comprises a fluorescent moiety and the second reporter group of probe 1 comprises a fluorescence quenching moiety;
(b) the first reporter group of probe 1 comprises a donor fluorescent moiety and the second reporter group of probe 1 comprises an acceptor fluorescent moiety capable to fluoresce upon transfer of energy from the donor fluorescent moiety;
(c) the first reporter group of probe 1 comprises an acceptor fluorescent moiety capable to fluoresce upon transfer of energy from a donor fluorescent moiety and the second reporter group of probe 1 comprises a donor fluorescent moiety;
(d) the first reporter group of probe 1 comprises a fluorescent moiety and the first reporter group of probe 2 comprises a fluorescence quenching moiety;
(e) the first reporter group of probe 1 comprises a fluorescence quenching moiety and the first reporter group of probe 2 comprises a fluorescent moiety;
(f) the first reporter group of probe 1 comprises a donor fluorescent moiety and the first reporter group of probe 2 comprises an acceptor fluorescent moiety capable to fluoresce upon transfer of energy from the donor fluorescent moiety (embodiment (f) is illustrated in Fig. 4);
(g) the first reporter group of probe 2 comprises a donor fluorescent moiety and the first reporter group of probe 1 comprises an acceptor fluorescent moiety capable to fluoresce upon transfer of energy from the donor fluorescent moiety;
(h) the first reporter group of probe 1 comprises a fluorescence quenching moiety, the second reporter group of probe 1 comprises a fluorescent moiety, and the first reporter group of probe 2 comprises a fluorescent moiety, wherein both fluorescent moieties have different absorption and/or emission spectra (embodiment (h) is illustrated in Fig. 3);
(i) the first reporter group of probe 1 comprises a donor fluorescent moiety, the second reporter group of probe 1 comprises an acceptor fluorescent moiety capable to fluoresce upon transfer of energy from the donor fluorescent moiety, and the first reporter group of probe 2 comprises a fluorescence quenching moiety;
(j) the second reporter group of probe 1 comprises a donor fluorescent moiety, the first reporter group of probe 1 comprises an acceptor fluorescent moiety capable to fluoresce upon transfer of energy from the donor fluorescent moiety, and the first reporter group of probe 2 comprises a fluorescence quenching moiety;
(k) the first reporter group of probe 1 comprises a donor fluorescent moiety, the first reporter group of probe 2 comprises an acceptor fluorescent moiety capable to fluoresce upon transfer of energy from the donor fluorescent moiety and the second reporter group of probe 1 comprises a fluorescence quenching moiety;
(l) the first reporter group of probe 2 comprises a donor fluorescent moiety, the first reporter group of probe 1 comprises an acceptor fluorescent moiety capable to fluoresce upon transfer of energy from the donor fluorescent moiety, and the second reporter group of probe 1 comprises a fluorescence quenching moiety;
(m)the second reporter group of probe 1 comprises a donor fluorescent moiety, the first reporter group of probe 1 comprises an acceptor fluorescent moiety capable to fluoresce upon transfer of energy from the donor fluorescent moiety, and the first reporter group of probe 2 comprises an acceptor fluorescent moiety capable to fluoresce upon transfer of energy from the acceptor fluorescent moiety of the first reporter group of probe 1;
(n) the first reporter group of probe 1 comprises a donor fluorescent moiety, the second reporter group of probe 1 comprises an acceptor fluorescent moiety capable to fluoresce upon transfer of energy from the donor fluorescent moiety, and the first reporter group of probe 2 comprises an acceptor fluorescent moiety capable to fluoresce upon transfer of energy from the donor fluorescent moiety, wherein both acceptor fluorescent moieties have different absorption and/or emission spectra;
(o) the first reporter group of probe 2 comprises a donor fluorescent moiety, the first reporter group of probe 1 comprises an acceptor fluorescent moiety capable to fluoresce upon transfer of energy from the donor fluorescent moiety, and the second reporter group of probe 1 comprises an acceptor fluorescent moiety capable to fluoresce upon transfer of energy from the acceptor fluorescent moiety of the first reporter group of probe 1; or
(p) the second reporter group of probe 1 comprises a donor fluorescent moiety, the first reporter group of probe 2 comprises a donor fluorescent moiety, and the first reporter group of probe 1 comprises an acceptor fluorescent moiety capable to fluoresce upon transfer of energy from either of the two donor fluorescent moieties or from both of the two donor fluorescent moieties, wherein both donor fluorescent moieties have different absorption and/or emission spectra.

In a further aspect of the invention the fluorescent moiety is selected from the group consisting of fluorescein isothiocyanate (FITC), tetramethylrhodamine isothiocyanate (TRITC), phycoerythrin, Cy7, fluorescein (FAM), Cy3, Cy3.5, Texas Red, LightCycler-Red 640, LightCycler Red 705, tetramethylrhodamine (TMR), rhodamine derivative (ROX), hexachlorofluorescein (HEX), Cy5, Cy5.5, rhodamine 6G (R6G), the rhodamine derivative JA133, Alexa Fluor 488, Alexa Fluor 546, Alexa Fluor 633, Alexa Fluor 555, Alexa Fluor 647, fluorescent nanoparticles, and fluorescent transition metal complexes, such as europium.

In a further embodiment of the present invention the donor fluorescent moiety is selected from the group consisting of FITC, phycoerythrin, FAM, Cy3, Cy3.5, R6G, TMR, Alexa Fluor 488, and Alexa Fluor 555.

In a further embodiment of the present invention the acceptor fluorescent moiety capable to fluoresce upon transfer of energy from a donor fluorescent moiety is selected from the group consisting TRITC, Cy7, Cy3, Cy3.5, Texas Red, LightCycler-Red 640, LightCycler Red 705, TMR, ROX, HEX, Cy5, Cy5.5, the rhodamine derivative JA133, Alexa Fluor 546, Alexa Fluor 633, and Alexa Fluor 647.

In another aspect of the invention the fluorescence quenching moiety is 4-(4'-dimethyl-aminophenylazo)benzoic acid (Dabcyl), black hole quencher 1 (BHQ-1), black hole quencher 2 (BHQ-2), QSY-7, or QSY-35, or it is selected from the group of FRET pair acceptors consisting of TRITC, Cy7, Cy3, Cy3.5, Texas Red, LightCycler-Red 640, LightCycler Red 705, TMR, ROX, HEX, Cy5, Cy5.5, the rhodamine derivative JA133, Alexa Fluor 546, Alexa Fluor 633, and Alexa Fluor 647.

In another embodiment of the present invention the combination of the donor fluorescent moiety and the acceptor fluorescent moiety capable to fluoresce upon transfer of energy from the donor fluorescent moiety is selected from the pairs of fluorophores listed in Table 1.

**Table 1 Pairs of fluorophores which can be used in FRET detection systems**

| donor fluorescent moiety | acceptor fluorescent moiety |
|---|---|
| FITC | TRITC |
| Phycoerythrin | Cy7 |
| FAM | Cy7 |
| FAM | Cy3/Cy3.5 |
| FAM | Texas Red |
| FAM | LightCycler-Red 640 |
| FAM | LightCycler Red 705 |
| FAM | TMR |
| FAM | ROX |
| FAM | HEX |
| FAM | Cy5/Cy5.5 |
| Cy3/Cy3.5 | Cy5/Cy5.5 |
| R6G | Cy5/Cy5.5 |
| TMR | Cy5/Cy5.5 |
| TMR | JA133 |
| Alexa Fluor 488 | Alexa Fluor 546 |
| Alexa Fluor 488 | Alexa Fluor 633 |
| Alexa Fluor 555 | Alexa Fluor 647 |

It is contemplated that an acceptor fluorescent moiety of a FRET pair can function as the donor fluorescent moiety of another FRET pair, thus allowing multiplex detection systems. For example, one reporter group within the probe set of the present invention could comprise FAM which serves as donor fluorescence moiety; another reporter group within the probe set could comprise Cy3, Cy3.5 or TMR which serve as an acceptor fluorescent moiety capable to fluoresce upon transfer of energy from FAM and which serve at the same time as a donor fluorescence moiety; and a third reporter group within the probe set could comprise either Cy5 or Cy5.5 which serves as an acceptor fluorescent moiety capable to fluoresce upon transfer of energy from Cy3, Cy3.5 or TMR.

In another embodiment of the present invention the radioactive moiety is selected from the group consisting of ³²P, ³³P, ³⁵S, ¹²³I ¹⁸F, ³H, ¹⁴C, and complexes of radioactive metals.

In a further embodiment of the present invention the binding moiety is selected from the group consisting of an antigenic peptide, an antigenic small molecule, biotin, and a His-tag.

In a preferred embodiment of the invention the probe 2 molecules to which the first reporter group of probe 1 has been transferred are detected by the fluorescence signal of the first reporter group, by the quenching effect of the first reporter group, by the fluorescence signal of the first reporter group of probe 2, by binding of an optionally labelled antibody, by the radioactive signal, and/or by the binding of streptavidin.

In a particularly preferred method of the present invention a reporter group is transferred from the probe 1 to the probe 2 and/or from the probe 1 to the probe 3 and/or from the probe 3 to the probe 2 by a chemical reaction selected from the group consisting of
(a) substitution at the carbonyl carbon atom as depicted in reaction scheme (I): 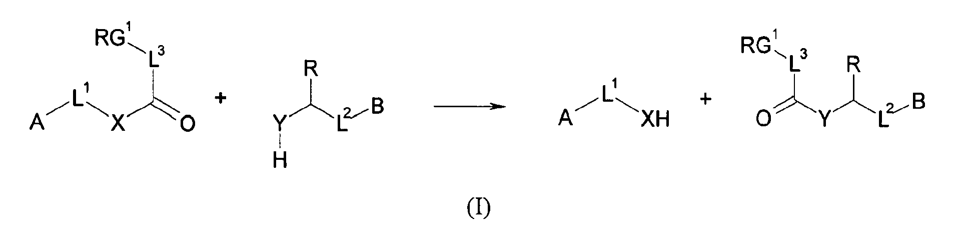 wherein
   - RG¹: is a first reporter group;
   - X: is S, O, Se, S-C(O), O-C(O), Se-C(O), or P⁺R¹R², wherein the C(O) group, if present, is bound to L¹; in especially preferred embodiments X is S or O;
   - Y: is NH, S, N-R⁴, HN-O, NR⁴-NR⁵, O, O-O, O-NH, S-S, S-O, PR³, P(OR³), Se, or a C nucleophile, wherein the S-O group is oriented in such that the O is bound to the carbon atom carrying the R group; in especially preferred embodiments Y is NH or S;
   - R: is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted;
   - R¹, R², and R³,: if present, are independently selected from the group consisting of aryl and alkyl; in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl;;
   - R⁴ and R⁵: if present, are independently from each other hydrogen; an alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl, heterocycloalkyl, aryl, heteroaryl, aralkyl, or a heteroaralkyl group, optionally substituted;
   - L¹: is a linker or a bond; L² is a linker or a bond; L³ is a linker or a bond; in a preferred embodiment L¹, L², or L³ is a bond;
   - A: comprises a region, which is complementary to a first region of the target nucleic acid sequence, and optionally at least a second reporter group which is linked to said region via a covalent bond or a linker; and
   - B: comprises a region, which is complementary to a second region of the target nucleic acid sequence, and optionally at least a first reporter group which is linked to said region via a covalent bond or a linker;
(b) substitution at the alkyl carbon atom as depicted in reaction scheme (II): 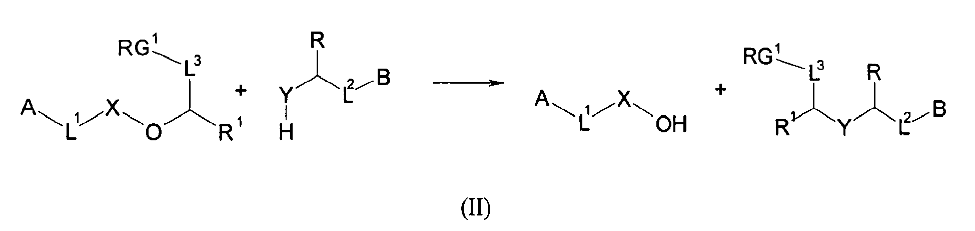 wherein
   - RG¹: is a reporter group;
   - X: is SO₂ or P⁺R²R³; in especially preferred embodiments X is SO₂;
   - Y: is NH, S, S-PO₃, N-R⁵, HN-O, NR⁵-NR⁶, O, O-O, ONH, S-S, S-O, PR⁴, P(OR⁴), Se, Se-PO₃, or a C nucleophile, wherein S-PO₃, Se-PO₃, S-O are oriented in such that the-PO₃ moiety or the O atom is bonded to the carbon atom carrying the R residue and S and Se are bonded to H before the reaction and to the carbon atom linked to L³ after the reaction; in especially preferred embodiments Y is NH, S, or S-PO₃;
   in especially preferred embodiments Y is NH, S, or S-PO₃;
   - R: is hydrogen, an alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl, heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted;
   - R¹: is -CN, -NO₂, -COOAlk, -H, -CHO, -COAlk;
   - R², R³, and R⁴: if present, are independently from each other aryl, in particular phenyl, and alkyl, in particular C₁-C₆ alkyl, e.g. C₁, C₂, C₃, C₄, C₅, or C₆ alkyl; optionally substituted;
   - R⁵ and R⁶,: if present, are independently from each other hydrogen; an alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert-*butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1*-iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group, optionally substituted;
   - L¹: is a linker or a bond; L² is a linker or a bond; L³ is a linker or a bond;
   - A: comprises a region, which is complementary to a first region of the target nucleic acid sequence, and optionally a second reporter group which is linked to said region via a covalent bond or a linker; and
   - B: comprises a region, which is complementary to a second region of the target nucleic acid sequence, and optionally a first reporter group which is linked to said region via a covalent bond or a linker;
(c) substitution at phosphate as depicted in reaction scheme (III): 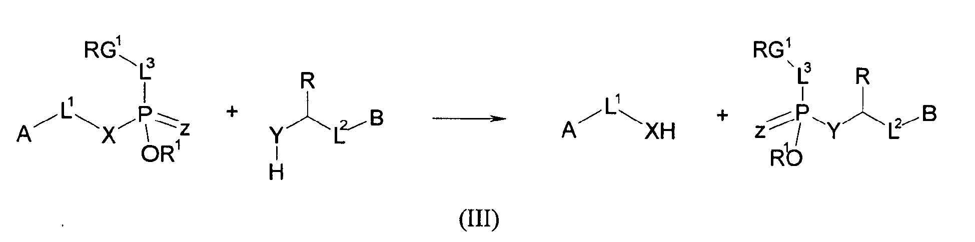 wherein
   - RG¹: is a reporter group;
   - X: is O, NR², or S;
   - Y: is O, NH, Se or S;
   - Z: is not present or O;
   - R and R¹: are independently from each other hydrogen, an a in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso-*propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinylaralkyl, or a heteroaralkyl group, optionally substituted; in especially preferred embodiments R¹ is hydrogen or methyl;
   - R²,: if present, is hydrogen; an alkyl in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group, optionally substituted; in especially preferred embodiments R² is hydrogen or methyl;
   - L¹: is a linker or a bond; L² is a linker or a bond; L³ is a linker or a bond;
   - A: comprises a region, which is complementary to a first region of the target nucleic acid sequence, and optionally a second reporter group which is linked to said region via a covalent bond or a linker; and
   - B: comprises a region, which is complementary to a second region of the target nucleic acid sequence, and optionally a first reporter group which is linked to said region via a covalent bond or a linker;
(d) Staudinger reaction as depicted in reaction scheme (IV): 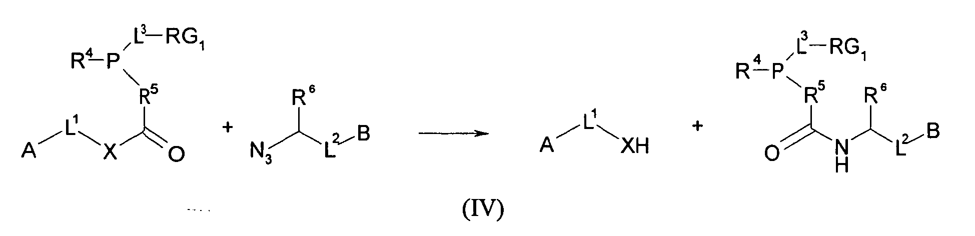 wherein RG₁ is a reporter group;
   - X: is O, S, Se, or NR³, wherein R³ is H or alkyl, in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert-*butyl, pentyl, hexyl;
   - R⁴: is hydrogen, an alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl, heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted; in preferred embodiments R⁴ is aryl or heteroaryl;
   - R⁵: is an alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group, optionally substituted; in preferred embodiments R⁵ is aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl;
   - R⁶: is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group, optionally substituted; in preferred embodiments R⁶ is hydrogen, C(O)-N-Alkyl, or -CH₃;
   - L¹: is a linker or a bond; L² is a linker or a bond; L³ is a linker or a bond;
   - A: comprises a region, which is complementary to a first region of the target nucleic acid sequence, and optionally a second reporter group which is linked to said region via a covalent bond or a linker; and
   - B: comprises a region, which is complementary to a second region of the target nucleic acid sequence, and optionally a first reporter group which is linked to said region via a covalent bond or a linker;
(e) Wittig reaction as depicted in reaction scheme (V): 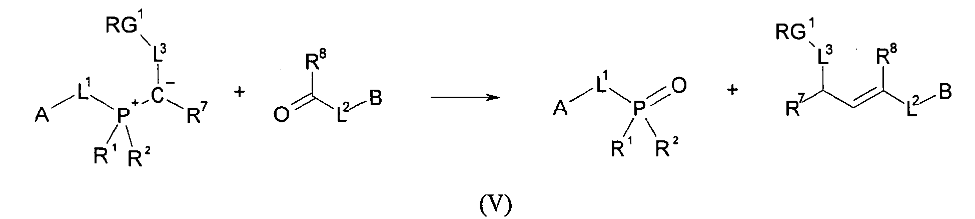
wherein RG₁ is a reporter group;
- R¹ and R²: are independently from each other selected from the group consisting of hydrogen, an alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl*,* pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; heteroaryl; aralkyl, and a heteroaralkyl group; optionally substituted; in preferred embodiments R¹ and R² are each independently from each other aryl, fluorinated alkyl, or -CH₂-aryl;
- R⁷: is C(O)N-alkyl, NO₂, CN, C(O)-alkyl, C(O)O-alkyl, aryl, heteroaryl, fluorinated alkyl; in preferred embodiments R⁷ is C(O)N-alkyl,
- R⁸: is hydrogen; CH=CH₂, aryl, in particular phenyl; alkyl in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert*-butyl, pentyl, hexyl; in preferred embodiments R⁸ is H or -CH₃;
- L¹: is a linker or a bond; L² is a linker or a bond; L³ is a linker or a bond; preferably L¹, L², and L³ is a bond;
- A: comprises a region, which is complementary to a first region of the target nucleic acid sequence, and optionally a second reporter group which is linked to said region via a covalent bond or a linker; and

- B: comprises a region, which is complementary to a second region of the target nucleic acid sequence, and optionally a first reporter group which is linked to said region via a covalent bond or a linker.

In a further embodiment of the present invention the one or more linkers, particularly L₁, L₂, and L₃, are selected from the group consisting of an alkyl, in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group, optionally substituted; aralkyl, and a heteroaralkyl group, optionally substituted.

In a preferred embodiment of the method of the present invention the probe 1 is represented by formula (VIII) 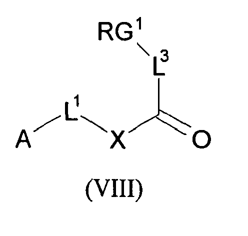 wherein
- RG₁: is a first reporter group;
- X: is S, O, Se, S-C(O), O-C(O), Se-C(O), or P⁺R¹R², wherein the C(O) group, if present, is bound to L¹; in preferred embodiments X is S or O;
- R¹ and R²,: if present, are independently selected from the group consisting of aryl and alkyl, preferably in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl;
- L¹: is a linker or a bond; L³ is a linker or a bond;
- A: comprises a region, which is complementary to a first region of the target nucleic acid sequence, and optionally a second reporter group which is linked to said region via a covalent bond or a linker;

In a preferred embodiment of the method of the present invention the probe 2 is represented by formula (VI) or formula (VII): 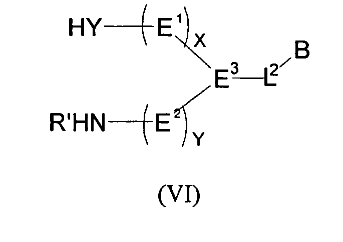 wherein
- E₁ and E₂: are independent of each other CHR", wherein R" is hydrogen; alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, heteroaryl, aralkyl, or a heteroaralkyl group; optionally substituted. In preferred embodiments R" is H, CH₃, C≡C-R³, CH=CR³R⁴.
- E³: is selected from the group consisting of alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert-*butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; heteroalkyl; heteroalkenyl; cycloalkyl, heterocycloalkyl, alicyclic system, aryl, in particular phenyl, naphtyl or anthracenyl; or heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; optionally substituted; and wherein E¹ and E² are attached to the same or to adjacent carbon and/or nitrogen atom(s); if E¹ and E² are attached to an alkyl, alkenyl, heteroalkyl, or heteroalkenyl, they are preferably attached to the same carbon or nitrogen atom and/or to a terminal residue of such an alkyl, alkenyl, heteroalkyl, or heteroalkenyl group;
- R': is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; in preferred embodiments R' is H or CH₃;
- R³ and R⁴,: if present, are independently from each other hydrogen; an alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1*-iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; in preferred embodiments R³ and R⁴ are independently from each other H or CH₃;
- L²: is a linker or a bond;
- B: comprises a region, which is complementary to a second region of the target nucleic acid sequence, and optionally a first reporter group which is linked to said region via a covalent bond or a linker;
- Y: is S or Se; and
one of X and Y is 1 and the other one is 0 or both X and Y are 1 or one of X and Y is 2 and the other one is 0;
or formula (VII): 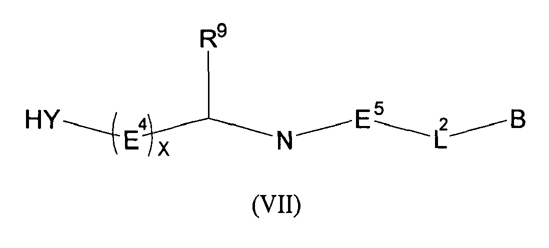 wherein
- E⁴: in each instance is independently CHR", wherein R" is hydrogen, alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1*-iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; optionally substituted;
- E⁵: is CHR"' or CR"', wherein R"' is hydrogen, alkyl, in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2*-iso-*propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; optionally substituted;
- R⁹: is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; optionally substituted; preferably R⁹ is hydrogen;
- or R⁹ and R"': are taken together to form a heterocycloalkyl, alicylic system or heteroaryl; preferably pyridinyl, optionally substituted;
- L²: is a linker or a bond;
- B: comprises a region, which is complementary to a second region of the target nucleic acid sequence, and optionally a first reporter group which is linked to said region via a covalent bond or a linker;
- Y: is S or Se; and
- x: is 1 or 2.

In a preferred embodiment of this method of the present invention the probe 2 is represented by formula (XI): 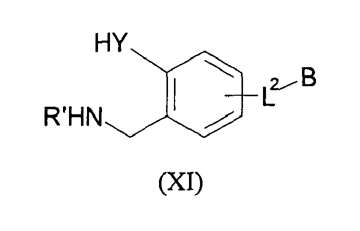 wherein
- L²: is a linker or a bond; and
- B: comprises a region, which is complementary to a second region of the target nucleic acid sequence, and optionally a first reporter group which is linked to said region via a covalent bond or a linker; and
- R': is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; in preferred embodiments R' is H or CH₃; and
- Y: is S or Se.

In a further aspect of the present invention the distance between the first region and the second region of the target nucleic acid and/or the distance between the first and the third region of the target nucleic acid and/or the distance between the second and the third region of the target nucleic acid ranges from 0 to 10 nucleotides, i.e. said distance is 0,1, 2, 3, 4, 5, 6, 7, 8, 9, or 10 nucleotides.

In a further preferred embodiment the method of the present invention comprises the additional step of detecting the probe 1 and/or the probe 3.

In a further embodiment the method of the present invention comprises the following additional steps:
(iv) contacting a probe 1 and a probe 2 in a separate blank sample, which does not contain the target nucleic acid sequence, and
(v) detecting probe 1 molecules from which said first reporter group has been transferred and/or probe 2 molecules to which said first reporter group has been transferred.

In another preferred method of the present invention the probe 2 is immobilized on a stationary phase. This preferred method is illustrated in Fig. 5. This method preferably comprises a washing step carried out after step (ii) which removes the sample and the probe 1.

In a further embodiment of the present invention a third probe is added to the sample prior, during or after the transfer of the reporter group from the probe 1 to the probe 2.

In some of the embodiments where three probes are used, the second region of the target nucleic acid sequence is situated between the first region and the third region of the target nucleic acid, i.e. the three probes hybridize to the target nucleic acid in the orientation probe1-probe2-probe3, wherein both orientations with regard to the 5' to 3' orientation of the target nucleic acid may be possible. In further preferred embodiments, prior to the template catalyzed reaction, probe 1 comprises a first reporter group and probe 3 comprises another first reporter group and probe 2 comprises no reporter group. In these embodiments, the first reporter group of probe 1 is transferred by the template catalyzed reaction to probe 2, and also the first reporter group of probe 3 is transferred by the template catalyzed reaction to probe 2. The transfer of the first reporter group of probe 3 to probe 2 can occur prior to, simultaneously with or after the transfer of the first reporter group of probe 1 to probe 2. When the template catalyzed reactions are complete, probe 2 comprises two reporter groups: the former first reporter group of probe 1 and the former first reporter group of probe 3. In an especially preferred embodiment the first reporter group of probe 1 and the first reporter group of probe 3 interact with each other in order to effect a detectable (preferably quantifiable) signal. Such an interaction can be achieved, e.g. if the first reporter group of probe 1 is a fluorophor and the first reporter group of probe 3 is a fluorescence quencher or vice versa, or if the first reporter group of probe 1 is a fluorescence donor of a FRET pair and the first reporter group of probe 3 is a fluorescence acceptor of a FRET pair or vice versa. In the embodiments explained immediately above the selectivity of the assay will be greatly increased, because two reactions have to take place to generate the detectable signal, namely one transfer reaction from probe 1 to probe 2 and a second transfer reaction from probe 3 to probe 2. The person skilled in the art will be able to easily convert all particularly preferred embodiments listed above from (a) to (p) employing two probes to a system which employs three probes. More precisely, all particularly preferred embodiments, wherein the probe 2 comprises a first reporter group (i.e. particularly preferred embodiments (d) to (p)) can easily be converted to a probe set of three probes, if the probe 2 described in embodiments (d) to (p) is replaced by a probe 2 without a reporter group and if a probe 3 is introduced into the probe set which comprises a transferable reporter group which after transfer of said transferable reporter group of probe 3 to probe 2 generates the probe 2 described in the corresponding embodiment (d) to (p). It is further contemplated that in such embodiments employing three different probes within a probe set that probe 1 and/or probe 3 comprise additional non-transferable reporter groups which may or may not interact with the transferable reporter group of the respective probe.

In preferred embodiments of the present invention the target nucleic acid is DNA or RNA. In further preferred embodiments the target nucleic acid is a prokaryotic, viral or eukaryotic nucleic acid. In an especially preferred embodiment of the present invention the target nucleic acid contains a single nucleotide polymorphism (SNP). In another embodiment the target nucleic acid is a splice variant of a naturally occurring nucleic acid.

In a preferred embodiment of the present invention several probe sets are used comprising the same first probe and two or more second probes, i.e. 3, 4, 5, 6, 7, 8, 9, or 10 different second probes, which differ in one or more nucleotides. Preferably, the transfer of the first reporter group to each of the one or more second probes will lead to a distinct signal. For example, in an embodiment using a first probe and four second probes, which all differ in one nucleotide, and which are all labelled with a different fluorophore, the transfer of a quenching moiety from the first probe will only suppress fluorescence from the fluorophore attached to the second probe having the correct complementary sequence. Thus, in one reaction it can be determined which out of four nucleotides reside at a given position of a target nucleotide. In a similar embodiment two or more first probes, e.g. 2, 3, 4, 5, 6, 7, 8, 9, or 10 different first probes, which are all directed against the same target sequence but differing in one or more nucleotides are used together with one second probe in two or more probe sets. Again the various probes are labelled in such that it is possible to determine which of the probes hybridize adjacent to each other. For example, four first probes differing in one nucleotide could be used all carrying a quencher moiety and each carrying a different fluorophor. Upon transfer of the quencher to the second probe only the fluorescence of those first probe would become visible capable of hybridizing adjacent to the second probe. Accordingly, in a preferred embodiment of the method of the present invention 2, 3, 4, 5, 6, 7, 8, 9, 10 or more first probes and/or second probes which differ in nucleotide sequence and/or reporter group are used in one probe set. This method is particularly suitable to determine if one of two or more known mutations are present in a target nucleic acid. The reporter groups can be selected in such that the presence of the one mutation leads to a different signal than the presence of the other mutation. Someone of skill in the art could determine suitable combinations of reporter groups in such sets to arrive at the desired result.

The present invention also provides a kit for detecting at least one target nucleic acid sequence in a sample comprising one probe set for each target nucleic acid sequence, the probe set comprising:
(a) a probe 1 having the structure (VIII) 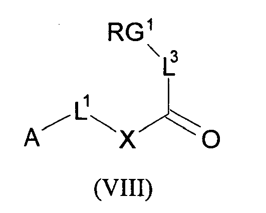 wherein
   - RG₁: is a first reporter group;
   - X: is S, O, Se, S-C(O), O-C(O), Se-C(O), or P⁺R¹R², wherein the C(O) group, if present, is bound to L¹; in especially preferred embodiments X is S or O;
   - R¹ and R²,: if present, are independently selected from the group consisting of aryl and alkyl;
   - L¹: is a linker or a bond; L³ is a linker or a bond;
   - A: comprises a region, which is complementary to a first region of the target nucleic acid sequence, and optionally a second reporter group which is linked to said region via a covalent bond or a linker;
   and
(b) a probe 2 having the structure (IX) or (X) 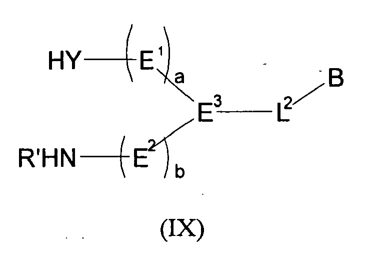 wherein
   - E¹ and E²: are independent of each other CHR", R" being a hydrogen, alkyl, alkenyl, alkynyl, cycloalkyl, heterocycloalkyl, aryl, heteroaryl, aralkyl, or a heteroaralkyl group; in preferred embodiments R" is H, CH₃, C≡C-R³, CH=CR³R⁴;
   - E³: is selected from the group consisting of alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert-*butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; heteroalkyl; heteroalkenyl; cycloalkyl, heterocycloalkyl, alicyclic system, aryl, in particular phenyl, naphtyl or anthracenyl; or heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quipazolinyl, quinolinyl, 1,2,3-benzotriazinyl, or 1,2,4-benzotriazinyl; optionally substituted; and wherein E¹ and E² are attached to the same or to adjacent carbon and/or nitrogen atom(s); if E¹ and E² are attached to an alkyl, alkenyl, heteroalkyl, or heteroalkenyl, they are preferably attached to the same carbon or nitrogen atom and/or to a terminal residue of such an alkyl, alkenyl, heteroalkyl, or heteroalkenyl group;
   - R': is hydrogen, an alkyl, alkenyl, alkynyl, cycloalkyl, heterocycloalkyl, aryl, heteroaryl, aralkyl, or a heteroaralkyl group; in preferred embodiments R' is H or CH₃;
   - R³ and R⁴,: if present, are independently from each other hydrogen; an alkyl, in particular C₁-C₆ alkyl, e.g. C₁, C₂, C₃, C₄, C₅, or C₆ alkyl, preferably methyl, ethyl, propyl, *iso-*propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, *1-iso-*propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; ; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted; in preferred embodiments R³ and R⁴ are independently from each other H or CH₃;
   - Y: is S or Se;
   - L²: is a linker or a bond;
   - B: comprises a region, which is complementary to a second region of the target nucleic acid sequence, and optionally a first reporter group which is linked to said region via a covalent bond or a linker;
   and one of a and b is 1 and the other one is 0 or both a and b are 1 or one of a and b is 2 and the other one is 0;
   or 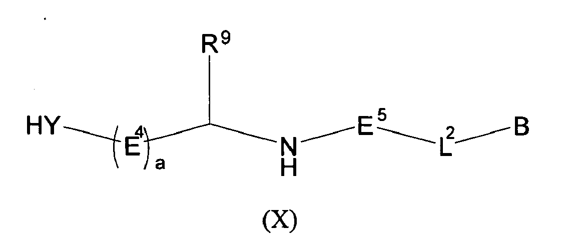 wherein
   - E⁴: in each instance is independently CHR", wherein R" is hydrogen, an alkyl, in particular C₁-C₆ alkyl, e.g. C₁, C₂, C₃, C₄, C₅, or C₆ alkyl, preferably methyl, ethyl, propyl, *is*o-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; ; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted; in preferred embodiments R" is hydrogen or CH₃;
   - E⁵: is CHR"' or CR"', wherein R"' is hydrogen, an alkyl, in particular C₁-C₆ alkyl, e.g. C₁, C₂, C₃, C₄, C₅, or C₆ alkyl, preferably methyl, ethyl, propyl, *iso*-propyl, butyl, *iso-*butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2*-iso-*propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; ; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted; in preferred embodiments R"' is H;
   - R⁹: is hydrogen; an alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl*, tert*-butyl*,* pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl; or a heteroaralkyl group; optionally substituted; or
   - R⁹ and R'": are taken together to form a heterocycloalkyl, alicylic system or heteroaryl; optionally substituted; preferably a pyridinyl;
   - Y: is S or Se;
   - L²: is a linker or a bond;
   - B: comprises a region, which is complementary to a second region of the target nucleic acid sequence, and optionally a first reporter group which is linked to said region via a covalent bond or a linker; and
   - a: is 1 or 2.
   wherein the second region of the target nucleic acid sequence is adjacent to the first region of the target nucleic acid.

In a preferred embodiment of the kit of the present invention the probe 2 is represented by formula (XI): 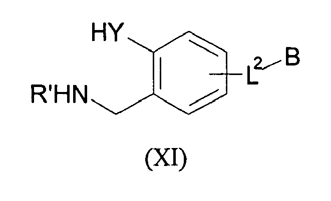 wherein
- L²: is a linker or a bond; and
- B: comprises a region, which is complementary to a second region of the target nucleic acid sequence, and optionally a first reporter group which is linked to said region via a covalent bond or a linker; and
- R': is hydrogen; alkyl, in particular C₁-C₆ alkyl, e.g. C₁, C₂, C₃, C₄, C₅, or C₆ alkyl, preferably methyl, ethyl, propyl, *iso*-propyl, butyl, *iso*-butyl, *tert*-butyl, pentyl, hexyl; alkenyl, in particular C₂-C₆ alkenyl, e.g. C₂, C₃, C₄, C₅, or C₆ alkenyl, preferably ethenyl, 1-propenyl, 2-propenyl, 1-*iso*-propenyl, 2-*iso*-propenyl, 1-butenyl, 2-butenyl, 3-butenyl; alkynyl, in particular C₂-C₆ alkynyl, e.g. C₂, C₃, C₄, C₅, or C₆ alkynyl; cycloalkyl; heterocycloalkyl; aryl, in particular phenyl, naphtyl or anthracenyl; heteroaryl, in particular furanyl, thienyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothienyl, 2-benzothienyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, 2,3-benzodoazinyl, quinoxalinyl, quinazolinyl, quinolinyl, 1,2,3-benzotriazinyl, or 1,2,4-benzotriazinyl; aralkyl, or a heteroaralkyl group; in preferred embodiments R' is H or CH₃; and
- Y: is S or Se.

All of the aspects and embodiments listed within this specification as aspects, embodiments or preferred embodiments of methods of the present invention are also preferred embodiments of the kits of the present invention, except for those instances where it is explicitly stated otherwise or where those aspects, embodiments or preferred embodiments cannot be applied to kits. This last statement applies also vice versa, i.e. all of the aspects and embodiments listed within this specification as aspects, embodiments or preferred embodiments of kits of the present invention are also preferred embodiments of the methods of the present invention, except for those instances where it is explicitly stated otherwise or where those aspects, embodiments or preferred embodiments cannot be applied to methods. In those instances where it is not explicitly stated within this specification whether an aspect, embodiment or preferred embodiment of the invention applies to methods or kits of the invention, it applies to both, except for those instances where the aspect, embodiment or preferred embodiment cannot be applied to either methods or kits.

Although some of the preferred embodiments of kits of the invention, which are explained in the following, have already been presented as aspects or preferred embodiments of the methods of the present invention, they are nevertheless repeated here to further illustrate the invention.

In a preferred kit of the present invention the regions which are complementary to the first region of the target nucleic acid sequence or to the second region of the target nucleic acid sequence are independently from each other selected from the group consisting of DNA, RNA, PNA, PS-DNA, OMe-RNA, MOE-RNA, NP, FANA, LNA, MF, CeNA and tcDNA.

In a further preferred embodiment of the kit of the present invention the probe 1 comprises a second reporter group.

In a further preferred embodiment of the kit of the present invention the probe 2 comprises a first reporter group. Said first reporter group of probe 2 is preferably different from the first reporter group of probe 1 and more preferably it is also different from any other reporter group which probe 1 may comprise.

In a preferred kit of the present invention the probe set further comprises a probe 3, which comprises a region, which is complementary to a third region of the target nucleic acid, probe 3 optionally comprising a first reporter group, wherein said third region is adjacent to the first region of the target nucleic acid or to the second region of the target nucleic acid. In a preferred embodiment of this kit of the present invention the region which is complementary to the third region of the target nucleic acid sequence is selected from the group consisting of DNA, RNA, PNA, PS-DNA, OMe-RNA, MOE-RNA, NP, FANA, LNA, MF, CeNA and tcDNA.

In a preferred kit of the present invention the one or more reporter groups are selected from the group consisting of a fluorescent moiety, a quenching moiety, a donor fluorescent moiety, an acceptor fluorescent moiety capable to fluoresce upon transfer of energy from a donor fluorescent moiety, a radioactive moiety, a binding moiety, wherein the one or more reporter groups are chosen in such that the transfer of a first reporter group of probe 1 and/or the transfer of a second reporter group of the probe 1 allows detection of probe 2 and/or probe 3.

In a further aspect the present invention relates to the use of the methods and/or the kits of the invention for determining the sequence of a target nucleic acid. The invention further relates to the use of the methods and/or the kits of the invention for the detection of at least one single nucleotide polymorphism (SNP) in at least one target nucleic acid. In a further embodiment, the use of the methods and/or the kits of the invention is directed to the detection of at least one splice variant of a target nucleic acid. These SNPs or these splice variants can be an indication for a disease, such as a hereditary disease, or they can be a tumor marker, or they may be used in pedigree analyses. In another embodiment, the kits and methods of the present invention are used in positional cloning, preferably by detecting genetic markers.

In a further embodiment of the present invention the methods and/or kits of the present invention are used for the detection of at least one target nucleic acid from at least one pathogenic organism, preferably a virus, a bacterium, or a fungus. In a further embodiment of the present invention the methods and/or kits of the present invention are used for the detection of at least one target nucleic acid from at least one organism causing allergic reactions, such as cereals, peanut, hazelnut.

In a further aspect of the present invention the methods and kits of the present invention can be used for the sequencing of nucleic acids. Techniques in which the methods and kits of the present invention can be employed include without limitation sequencing by hybridization (SBH) (Fedrigo O. and Naylor G. (2004) Nucleic Acids Res. 32(3), 1208-1213; Zhang J.H. et al (2003) Bioinformatics 19(1) 14-21; Drmarnac R. et al (2002) Adv. Biochem. Eng. Biotechnol. 77, pp. 75-101) and sequencing by hybridization to oligonucleotide microchips (SHOM) (Yershov, G. et al. (1996) Proc. Natl. Acad. Sci. USA 93(10) 4913-4918). The person skilled in the art will know which modifications, if any, will be necessary to employ the methods and kits of the present invention in SBH and SHOM.

A non-limiting example on how the methods and kits of the present invention can be used in sequencing by hybridization is explained in the following: An oligonucleotide chip is constructed which contains 16384 different compartments. In each compartment a different probe 2 is immobilized, more specifically the different probes 2 differ only in their complementary region. Each probe 2 comprises a different heptanucleotide sequence (4⁷ = 16384). Then the sample with the target nucleic acid and a mixture of probe 1 molecules are added to each compartment. The mixture of probe 1 molecules comprises every combination of dekanucleotide sequences with the exception that the terminal nucleotide of each probe 1 molecule is A, i.e. the nucleotide which is the nucleotide most adjacent to probe 2 when both probe 1 and probe 2 hybridize to the target nucleic acid is A (4⁹ combinations = 262144 different probe 1 molecules). Furthermore, all probe 1 molecules comprise the same reporter group, preferably said same reporter group comprises a fluorescent moiety. Then the chip is exposed to conditions which allow the transfer of the reporter group from the probe 1 to probe 2, if probe 1 and probe 2 hybridize to adjacent regions of the target DNA. After the reaction the mixture of probe 1 molecules and the sample are removed from the chip. In this detection round all octanucleotide sequences within the target nucleic acid sequence which start with A are detected and labelled with the reporter group. Said octanucleotide sequences can be detected by means well known to the skilled person, and the different octanucleotide sequences can be assembled using computer programs to yield a long complete nucleic acid sequence. Optionally, the process can be repeated in an unused chip of heptanucleotide sequences or even in the same chip with a mixture of probe 1 molecules comprising every combination of dekanucleotide sequences with the exception that the terminal nucleotide of each probe 1 molecule is C. In this mixture, the probe 1 molecules preferably comprise a different reporter group than the one that was used in the first detection round. In this second detection round all octanucleotide sequences within the target nucleic acid sequence which start with C are detected and labelled with a reporter group. Preferably, the data obtained from the first detection round, from this second detection round and from the third and fourth detection round (using a mixture of probe 1 molecules with G as terminal nucleotide and with T as terminal nucleotide, respectively; and preferably with a different third and different fourth reporter group, respectively) are combined and assembled into the complete nucleic acid sequence using a computer program.

In a further aspect of the present invention any probe of the methods or kits of the present invention may be protected by a protecting group in such a way that the transfer of the reporter group cannot occur as long as the protecting group is bound to the probe. In a preferred embodiment of this aspect, the protecting group is a photolabile protecting group which leaves the probe molecule upon suitable irradiation. After removal of the protecting group a template catalyzed transfer reaction of a reporter group can occur. Examples of such photolabile protecting groups can be found e.g. in (Cameron et al. (1995) J. Chem. Soc., Chem. Commun. 923-924) and (Pirrung M. C. and Huang C.-Y. (1995) Tetrahedron Letters vol. 36, 33, 5883-5884). In an especially preferred embodiment of this aspect, the amino group of formula (VI) shown above is a secondary amino group and this secondary amino group of formula (VI) or the secondary amino group of formula (VII) shown above is protected by a methoxy-substituted benzoin group, preferably by a [(3',5'-dimethoxybenzoinyl)oxy]carbonyl group. For the chemical structure of this group and the mechanism underlying its removal see Cameron et al. (1995) J. Chem. Soc., Chem. Commun. 923-924. The person skilled in the art well knows that the removal of this protecting group by irradiation, preferably at about 350 nm, may necessitate an additional basic/nucleophilic washing step to cleave thioesters that may have formed in the vicinity of the protected amino group. The skilled person will know how to carry out such a washing step. A non-limiting example of a probe 2 protected by a [(3',5'-dimethoxybenzoinyl)oxy]carbonyl group is shown in the following formula (XII):

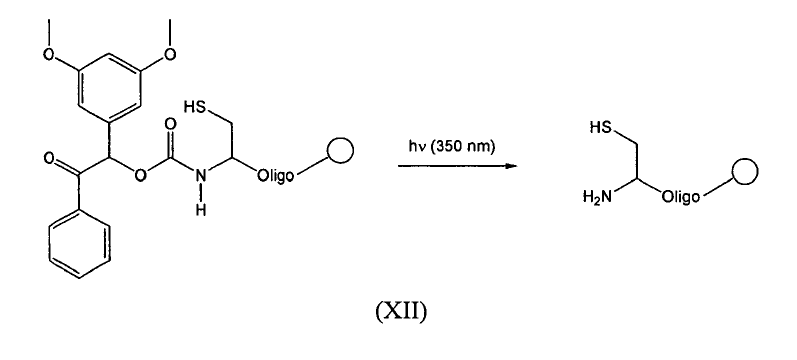

### BRIEF DESCRIPTION OF THE FIGURES

Fig. 1 illustrates the general principle of template catalyzed transfer reactions. Two probes bind to a single stranded target nucleic acid (shown in light gray). Upon binding of both probes a reporter group (shown as a circle filled in dark grey) is transferred from one probe to the other and the probes dissociate from the target nucleic acid.
Fig. 2 illustrates the principle of template catalyzed transfer reactions in an example where the principle of chemical ligation is modified to a transfer reaction. The donator probe and the acceptor probe are PNA probes and the target nucleic acid is a DNA. The reporter group (shown as circle filled in black) is linked to the donator probe via a thioester bond. The reporter group is first transferred to a thiol group of the acceptor probe, and then in an irreversible intramolecular reaction to an amino group of the acceptor probe.
Fig. 3 shows an embodiment of the invention, wherein the donator probe (probe 1) and the acceptor probe (probe 2) carry non-transferable reporter groups comprising different fluorescent moieties. The transferable reporter group (which can be transferred in a template catalyzed reaction) comprises a quenching moiety. Before the template catalyzed reaction takes place, the fluorescent moiety of the donator probe is quenched and the fluorescent moiety of the acceptor probe can fluoresce. After the template catalyzed reaction has taken place, the fluorescent moiety of the donator probe can fluoresce and the fluorescent moiety of the acceptor probe is quenched.
Fig. 4 shows an embodiment of the invention, wherein the acceptor probe (probe 2) carries a non-transferable reporter group comprising the acceptor moiety of a FRET pair (shown as circle filled in dark grey). The transferable reporter group (which can be transferred in a template catalyzed reaction) comprises the donator moiety of the FRET pair (shown as circle filled in light grey). Before the template catalyzed reaction takes place, the emission spectrum of the donator moiety of the FRET pair can be observed upon excitation at the excitation wavelength of the donator moiety. After the template catalyzed reaction has taken place, the emission spectrum of the donator moiety of the FRET pair can no longer be observed. Instead a fluorescence signal (a FRET signal) from the acceptor moiety of the FRET pair can be observed.
Fig. 5 shows an embodiment of the invention, wherein the acceptor probe (probe 2) is immobilized on a stationary phase. After the reporter group (shown as circle filled in light grey) of the donator probe (probe 1) has been transferred to the acceptor probe (probe 2) in a template catalyzed reaction, the target nucleic acid (template nucleic acid) and the donator probe (probe 1) are removed. Acceptor probe molecules which received a reporter group can be detected via a signal conferred by the reporter group, e.g. by a fluorescence signal.

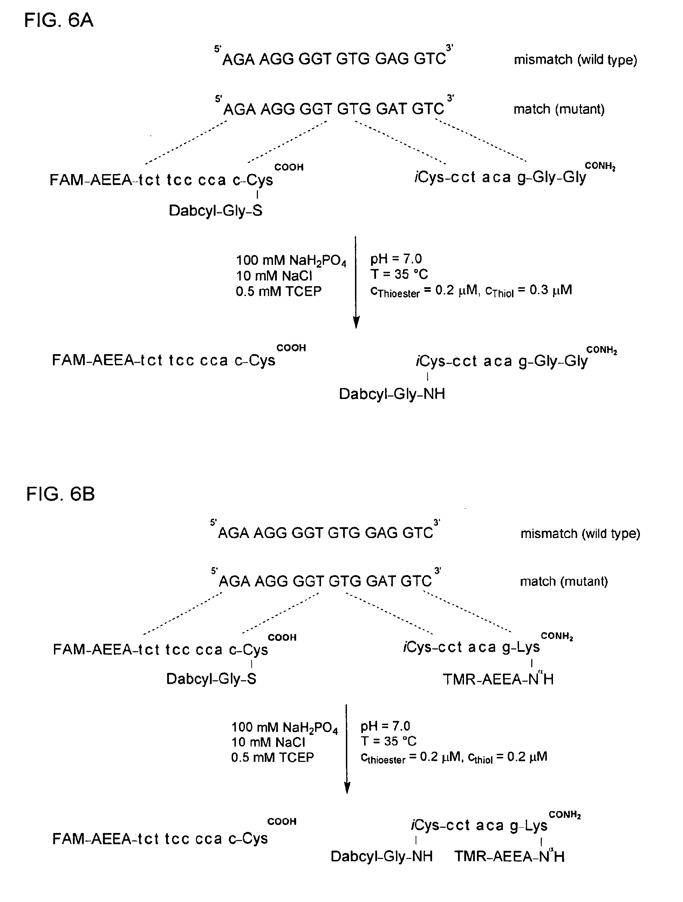

Fig. 6 illustrates the reaction conditions, the sequences of target DNAs and of the probes used in example 5 (= Fig. 6A) and example 6 (= Fig. 6B).

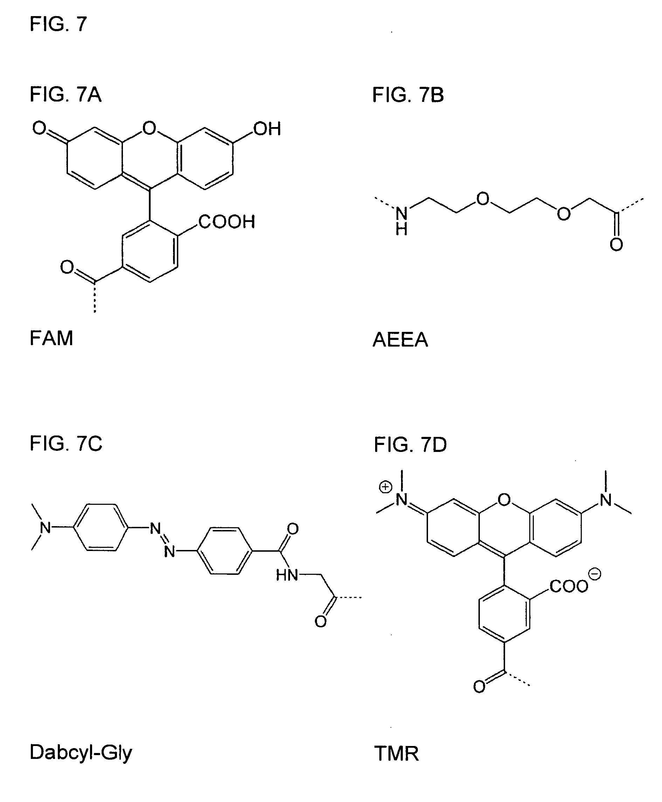

Fig. 7 shows the chemical formulae of the fluorescent moieties, of the quenching moiety and of a linker element used in examples 5 and 6, namely the chemical formulae of FAM (Fig. 7A), TMR (Fig. 7D), Dabcyl-Gly (Fig. 7C) and of AEEA (Fig. 7B).

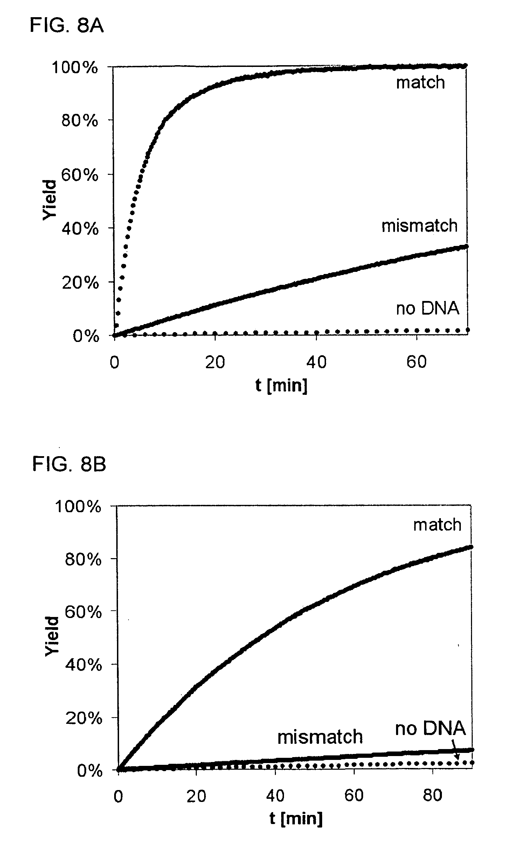

Fig. 8 shows the results of example 5. Fig. 8A shows the yield of the reaction over time in the presence of 1 eq. match DNA, 1 eq. mismatch DNA and in the absence of DNA.
Fig. 8B shows the yield of the reaction over time in the presence of 0.1 eq. match DNA, 0.1 eq. mismatch DNA, and in the absence of DNA.

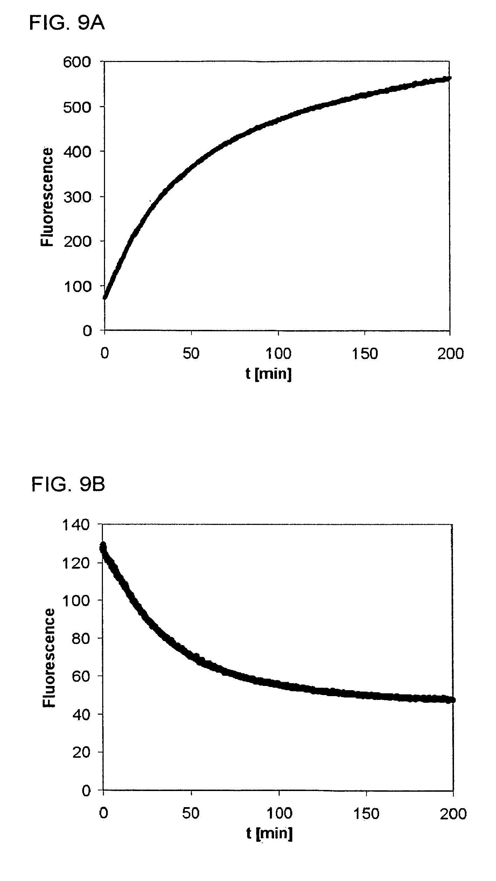

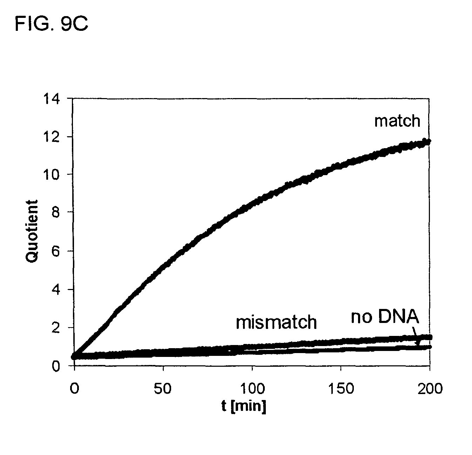

Fig. 9 shows the results of example 6. Fig. 9A shows the development over time of the fluorescence signals of FAM in the presence of 0.1 eq. match DNA. Fig. 9B shows the development over time of the fluorescence signals of TMR in the presence of 0.1 eq. match DNA. Fig. 9C shows the quotient of the time-dependent fluorescence signals of FAM to TMR in the presence of 0.1 eq. match DNA, in the presence of 0.1 eq. mismatch DNA, and in the absence of DNA.

### EXAMPLES / EXPERIMENTAL SECTION

### EXAMPLE 1

### General remarks on solid phase synthesis

The resins used in solid phase synthesis were loaded with the protected amino acids according to standard protocols (NovaBiochem Catalog, 2004/2005) (loading level about 0.15 mmol/g).

The resin was washed between coupling, capping, and deprotecting steps (1 mL each: 5 x *N,N* dimethylformamide (DMF), 5 x CH₂Cl₂, 5 x DMF). If the washing took place after treatment with trifluoroacetic acid (TFA), the first washing step was replaced by washing with 5 x CH₂Cl₂. The same applied to the last washing step, if treatment of the resin with TFA followed.

For the purification, the combined TFA phases were concentrated *in vacuo* to a volume of about 0.1 mL. The crude product was precipitated by addition of Et₂O (1 mL), spun down, and the supernatant was discarded. The pellet was resuspended in Et₂O, spun down, and the supernatant was again discarded. The crude product was dissolved in 0.3 mL of an aqueous solution (1 % CH₃CN, 0.1 % TFA in H₂O) and centrifuged.

The aqueous solution was purified using preparative RP-HPLC. In preparative HPLC, a *Polaris C18 A 5µ 250x100* (pore size 220 Å; manufacturer: Varian) was employed as separating column using a flow rate of 5 mL/min. In analytical HPLC, a *Polaris C18 A 5*µ *250x046* (pore size 220 Å; manufacturer: Varian) set to a temperature of 55 °C was employed as separating column using a flow rate of 1 mL/min. A binary mixture of A (98.9 % H₂O, 1 % acetonitrile, 0.1 % TFA) and B (98.9 % acetonitrile, 1 % H₂O, 0.1 % TFA) was used as the mobile phase (gradient I: from 3 % B to 30 % B in 30 min; gradient II from 3 % B to 60 % B in 30 min).After the eluent was removed *in vacuo* from the fractions containing the product, the product was dissolved in degassed H₂O.

### EXAMPLE 2

### Synthesis of FAM-AEEA-tct tcc cca c-Cys(S-Gly-Dabcyl)^{COOH}

The PNA sequence Fmoc-tc^{Bhoc} ttc^{Bhoe}c^{Bhoc}c^{Bhoc}c^{Bhoc}a^{Bhoc}c^{Bhoc} (Fmoc = fluorenylmethyloxycarbonyl) was built via a synthesizer (Jarikote, J.V. et al. (2005) Eur. J. Org. Chem. 3187-3195 ) on a Fmoc-Cys(Mmt)-TGA resin (amount loaded: 2 µmol). The subsequent synthesis was continued with half of the resin.

After shaking the resin twice for 5 min in DMF/piperidine (4:1, 0.5 mL each time), the resin was reacted twice with 10 equivalents Fmoc-AEEA-OH (final concentration about 0.02 M in DMF), 10 equivalents of benzotriazole-1-yl-oxy-tris-pyrrolidino-phosphonium hexafluorophosphate (PyBOP), and 25 equivalents of *N*-methylmorpholine (NMM). Subsequently, the resin was first shaken in pyridine/Ac₂O (10:1, 1 mL) for 5 min, and then twice in DMF/piperidine (4:1, 0.5 mL each time) for 5 min. The resin was reacted twice with 10 eq. 6-carboxyfluorescein (FAM-OH; final concentration about 0.02 M in DMF), 10 eq. PyBOP, and 20 eq. NMM each for 1 h. After shaking in pyridine/Ac₂O (10:1, 1 mL) for 5 min, the resin was treated with DMF/piperidine (4:1, 1 mL).

The resin was treated three times with CH₂Cl₂/TIS/TFA (93:5:2, 1 mL each time) for 10 min, and subsequently shaken 6 times with 5 eq. Dabcyl-Gly-OH (final concentration about 0.02 M in DMF), 4.5 eq. PyBOP and 12.5 eq. NMM each for 1 h. For release of the product the resin was shaken with TFA/H₂O/*m*-cresol (18:1:1, 0.6 mL) and subsequently extracted 4 times with TFA (0.2 mL each time).

The OD₂₆₀ of the product was 3.52 corresponding to a yield of 35.0 nmol or 3.5 %. relative to the loading level of the Fmoc-Cys(Mmt)-TGA resin. The (m/z) quotient in the MALDI-TOF/MS analysis for the [M+H]⁺ form was calculated to be 3515.4, and a value of 3515.8 was detected. The retention time (*t*_{R}) in HPLC was found to be 19.2 min when applying gradient II.

### EXAMPLE 3

### Synthesis of iCys-cct aca g-Gly-Gly-^{CONH}2

The protecting group Fmoc of the Fmoc-Gly-MBHA resin (loading level: 2.5 µmol) was removed by treatment with DMF/piperidin (4:1, 1 mL). The PNA peptide sequence was subsequently built according to the Boc strategy, and the product was separated from the resin (Ficht et al. (2005) Chembiochem. 6, 2098-2103).

The OD₂₆₀ of the product was 88.9 corresponding to a yield of 1.34 µmol or 54 % relative to the loading level of the Fmoc-Gly-MBHA resin. The (m/z) quotient in the MALDI-TOF/MS analysis for the [M+H]⁺ form was calculated to be 2095.8, and a value of 2095.6 was detected. The retention time (*t_{R}*) in HPLC was found to be 9.0 min when applying gradient I.

### EXAMPLE 4

### Synthesis of iCys-cct aca g-Lys(N^{α}-Gly-Dabcyl^{)CONH}₂

The protecting group Fmoc of the Fmoc-Lys(Boc)-MBHA resin (loading level 2 µmol) was removed by treatment with DMF/piperidin (4:1, 1 mL). Subsequently, the resin was reacted twice with 10 eq. Fmoc-AEEA-OH (final concentration 0.1 M in DMF), 10. eq. PyBOP, and 25 eq. NMM each for 1 h. Thereafter, it was shaken in pyridine/Ac₂O (10:1, 1 mL) for 5 min, and then twice in DMF/piperidine (4:1, 0.5 mL each time) for 5 min. The resin was then reacted with 4 eq. TMR-OH (final concentration 0.05 M in DMF), 4 eq. PyBOP, and 10 eq. NMM for 1 h. The PNA peptide sequence was subsequently built according to the Boc strategy, and the product was separated from the resin (Ficht et al. (2005) Chembiochem. 6, 2098-2103).

The OD₂₆₀ of the product was 30.4 corresponding to a yield of 0.407 µmol or 20 % relative to the loading level of the Fmoc-Lys(Boc)-MBHA resin. The (m/z) quotient in the MALDI-TOF/MS analysis for the [M+H]⁺ form was calculated to be 2668.7, and a value of 2669.3 was detected. The retention time (*t_{R}*) in HPLC was found to be 16.4 min when applying gradient II.

### EXAMPLE 5

### Template catalyzed transfer of a Dabcyl-Gly group to an unlabelled probe

A sequence encoding a section of the Ras-protein was selected as target DNA. The two-fold labelled PNA thioester probe 1 (donator probe) and the unlabelled iso-cysteine probe 2 (acceptor probe) were synthesized at the solid phase in high yields.

The donator probe 1 is complementary to a constant sequence section of the DNA. The point mutation shown from G → T is located in the middle of the segment, which is bound by acceptor probe 2. Probe 2 is complementary to the mutant DNA (match) and exhibits a single base mismatch with the wild-type DNA (Fig. 6A).

The yield, the initial rate and the selectivity of the template catalyzed transfer reaction was studied at 35°C. In the following experiments, all conditions were kept constant, except for the analyte concentrations (match or mismatch DNA). These concentrations were adjusted in relation to the concentration of the donator probe 1 (0.2 µM) to ratios of 1:1, 1:10, 1:100, and 1:1000. The reaction with the match DNA resulted in the turnover rates shown in table 2.

**Table 2: Turnover in the presence of match DNA (yield match - yield background)**

| | 1:10 | 1:100 | 1:1000 |
|---|---|---|---|
| Turnover for match DNA | 7.0 after 70 min | 68 after 1000 min | 3.2.10² after 1000 min |

The initial rate for the transfer reaction in the presence and in the absence (background) of DNA was also determined. These reactions resulted in the initial rates shown in table 3.

**Table 3: Initial rate [nM/s] for different analyte/probe ratios.**

| | 1:1 | 1:10 | 1:100 | 1:1000 | Background |
|---|---|---|---|---|---|
| match | 4.9 -10⁻¹ | 5.6-10⁻² | 7.5-10⁻³ | 2.1-10⁻³ | 6.1-10⁻⁴ |
| mismatch | 1.8-10⁻² | 2.6-10⁻³ | 1.0-10⁻³ | 6.8-10⁻⁴ | |

The selectivities of the assay with match DNA (mutant) as compared to the background reaction and as compared to the mismatch DNA (wild-type) are shown in table 4.

**Table 4: Selectivity**

| | 1:1 | 1:10 | 1:100 | 1:1000 |
|---|---|---|---|---|
| match / background | 8.1·10² | 91 | 12 | 3.5 |
| match / mismatch | 27 | 21 | 7.3 | 3.2 |

### EXAMPLE 6

### Template catalyzed transfer of a Dabcyl-Gly group to a probe labelled with TMR

The sequence of the target DNA and the PNA sequences of probe 1 ("donator probe" or "thioester probe") and of probe 2 ("acceptor probe" or "thiol probe") were the same as in Example 5. Reaction conditions and probe 1 were the same as in Example 5. Probe 2 (the acceptor probe or "thiol probe") was replaced by a probe which had the identical PNA sequence as probe 2 in Example 5, but carried an additional TMR group (see Fig. 6B). The concentration of probe 2 was 0.2 µM. The concentration of probe 1 remained unchanged.

The reaction was monitored via fluorescence spectroscopy (FAM: excitation wavelength 485 nm, emission wavelength 525 nm; TMR: excitation wavelength 558 nm, emission wavelength 585 nm). A complete reaction leads to an increase of the FAM fluorescence by the factor 10 and a decrease of the TMR fluorescence by the factor 3. Fig. 9A and 9B show the development over time of the fluorescence signals of FAM (Fig. 9A) and TMR (Fig. 9B) in the presence of 0.02 µM match DNA (i.e. DNA concentration is 1:10 of each probe).

The quotient of the time-dependent fluorescence signals of FAM to TMR in the presence of 0.1 eq. match DNA, in the presence of 0.1 eq. mismatch DNA and in the absence of DNA was calculated and is shown in Fig. 9C. When applying this technique a significant increase in the sensitivity of the assay is observed. Moreover, comparing the time-dependent fluorescence signals allows a distinct discrimination between match DNA and mismatch DNA. A turnover rate of more than 7 (theoretical maximum: 10) was observed in the presence of 0.1 eq. match DNA after 200 min.

## Claims

1. A method for detecting at least one target nucleic acid sequence in a sample comprising the steps of:
(i) contacting the sample with at least one probe set for each target nucleic acid sequence, the probe set comprising:
(a) a probe 1 comprising a first reporter group, which is capable of being transferred to a probe 2, and a region, which is complementary to a first region of the target nucleic acid sequence, and
(b) a probe 2 comprising a region, which is complementary to a second region of the target nucleic acid sequence and a moiety which is capable of receiving said first reporter group when both probe 1 and probe 2 hybridize to the target nucleic acid,
wherein said second region of the target nucleic acid sequence is adjacent to the first region of the target nucleic acid;
(ii) exposing the sample to conditions which lead to the transfer of the first reporter group of probe I to probe 2; and
(iii) detecting probe 2 molecules to which said first reporter group has been transferred.

2. The method of claim 1, wherein the region of the probe 1 complementary to a first region of the target nucleic acid is selected from the group consisting of DNA, RNA, PNA, PS-DNA, OMe-RNA, MOE-RNA, NP, FANA, LNA, MF, CeNA and tcDNA.

3. The method of claim 1 or 2, wherein probe 1 comprises a second reporter group.

4. The method of any one of claims 1 to 3, wherein the region of probe 2 complementary to a second region of the target nucleic acid is selected from the group consisting of DNA, RNA, PNA, PS-DNA, OMe-RNA, MOE-RNA, NP, FANA, LNA, MF, CeNA and tcDNA.

5. The method of any one of claims 1 to 4, wherein probe 2 comprises a first reporter group.

6. The method of any one of claims 1 to 5, wherein the probe set further comprises a probe 3, which comprises a region which is complementary to a third region of the target nucleic acid, probe 3 optionally comprising a first reporter group, wherein said third region is adjacent to the first region of the target nucleic acid or to the second region of the target nucleic acid.

7. The method of any one of claims 1 to 6, wherein the one or more reporter groups are selected from the group consisting of a fluorescent moiety, a quenching moiety, a donor fluorescent moiety, an acceptor fluorescent moiety capable to fluoresce upon transfer of energy from a donor fluorescent moiety, a radioactive moiety, a binding moiety, wherein the one or more reporter groups are chosen in such that the transfer of a first reporter group of probe 1 and/or the transfer of a second reporter group of the probe 1 allows detection of probe 2 and/or probe 3.

8. The method of any of claims 1 to 7, wherein
(a) the first reporter group of probe 1 comprises a fluorescent moiety and the second reporter group of probe 1 comprises a fluorescence quenching moiety;
(b) the first reporter group of probe 1 comprises a donor fluorescent moiety and the second reporter group of probe 1 comprises an acceptor fluorescent moiety capable to fluoresce upon transfer of energy from the donor fluorescent moiety;
(c) the first reporter group of probe 1 comprises an acceptor fluorescent moiety capable to fluoresce upon transfer of energy from a donor fluorescent moiety and the second reporter group of probe 1 comprises a donor fluorescent moiety;
(d) the first reporter group of probe 1 comprises a fluorescent moiety and the first reporter group of probe 2 comprises a fluorescence quenching moiety;
(e) the first reporter group of probe 1 comprises a fluorescence quenching moiety and the first reporter group of probe 2 comprises a fluorescent moiety;
(f) the first reporter group of probe 1 comprises a donor fluorescent moiety and the first reporter group of probe 2 comprises an acceptor fluorescent moiety capable to fluoresce upon transfer of energy from the donor fluorescent moiety;
(g) the first reporter group of probe 2 comprises a donor fluorescent moiety and the first reporter group of probe 1 comprises an acceptor fluorescent moiety capable to fluoresce upon transfer of energy from the donor fluorescent moiety;
(h) the first reporter group of probe 1 comprises a fluorescence quenching moiety, the second reporter group of probe 1 comprises a fluorescent moiety, and the first reporter group of probe 2 comprises a fluorescent moiety, wherein both fluorescent moieties have different absorption and/or emission spectra;
(i) the first reporter group of probe 1 comprises a donor fluorescent moiety, the second reporter group of probe 1 comprises an acceptor fluorescent moiety capable to fluoresce upon transfer of energy from the donor fluorescent moiety, and the first reporter group of probe 2 comprises a fluorescence quenching moiety;
(j) the second reporter group of probe 1 comprises a donor fluorescent moiety, the first reporter group of probe 1 comprises an acceptor fluorescent moiety capable to fluoresce upon transfer of energy from the donor fluorescent moiety, and the first reporter group of probe 2 comprises a fluorescence quenching moiety;
(k) the first reporter group of probe 1 comprises a donor fluorescent moiety, the first reporter group of probe 2 comprises an acceptor fluorescent moiety capable to fluoresce upon transfer of energy from the donor fluorescent moiety and the second reporter group of probe 1 comprises a fluorescence quenching moiety;
(l) the first reporter group of probe 2 comprises a donor fluorescent moiety, the first reporter group of probe 1 comprises an acceptor fluorescent moiety capable to fluoresce upon transfer of energy from the donor fluorescent moiety, and the second reporter group of probe 1 comprises a fluorescence quenching moiety;
(m) the second reporter group of probe 1 comprises a donor fluorescent moiety, the first reporter group of probe 1 comprises an acceptor fluorescent moiety capable to fluoresce upon transfer of energy from the donor fluorescent moiety, and the first reporter group of probe 2 comprises an acceptor fluorescent moiety capable to fluoresce upon transfer of energy from the acceptor fluorescent moiety of the first reporter group of probe 1;
(n) the first reporter group of probe 1 comprises a donor fluorescent moiety, the second reporter group of probe 1 comprises an acceptor fluorescent moiety capable to fluoresce upon transfer of energy from the donor fluorescent moiety, and the first reporter group of probe 2 comprises an acceptor fluorescent moiety capable to fluoresce upon transfer of energy from the donor fluorescent moiety, wherein both acceptor fluorescent moieties have different absorption and/or emission spectra;
(o) the first reporter group of probe 2 comprises a donor fluorescent moiety, the first reporter group of probe 1 comprises an acceptor fluorescent moiety capable to fluoresce upon transfer of energy from the donor fluorescent moiety, and the second reporter group of probe 1 comprises an acceptor fluorescent moiety capable to fluoresce upon transfer of energy from the acceptor fluorescent moiety of the first reporter group of probe 1; or
(p) the second reporter group of probe 1 comprises a donor fluorescent moiety, the first reporter group of probe 2 comprises a donor fluorescent moiety, and the first reporter group of probe 1 comprises an acceptor fluorescent moiety capable to fluoresce upon transfer of energy from either of the two donor fluorescent moieties or from both of the two donor fluorescent moieties, wherein both donor fluorescent moieties have different absorption and/or emission spectra.

9. The method of any one of claims 1 to 8, wherein the fluorescent moiety is selected from the group consisting of fluorescein isothiocyanate (FITC), tetramethylrhodamine isothiocyanate (TRITC), phycoerythrin, Cy7, fluorescein (FAM), Cy3, Cy3.5, Texas Red, LightCycler-Red 640, LightCycler Red 705, tetramethylrhodamine (TMR), rhodamine derivative (ROX), hexachlorofluorescein (HEX), Cy5, Cy5.5, rhodamine 6G (R6G), the rhodamine derivative JA133, Alexa Fluor 488, Alexa Fluor 546, Alexa Fluor 633, Alexa Fluor 555, Alexa Fluor 647, fluorescent nanoparticles, and fluorescent transition metal complexes.

10. The method of any one of claims 1 to 9, wherein the fluorescence quenching moiety is 4-(4'-dimethyl-aminophenylazo)benzoic acid (dabcyl), black hole quencher I (BHQ- 1), black hole quencher 2 (BHQ-2), QSY-7, or QSY-35, or it is selected from the group of FRET pair acceptors consisting of TRITC, Cy7, Cy3, Cy3.5, Texas⁻ Red, LightCycler-Red 640, LightCycler Red 705, TMR, ROX, HEX, Cy5, Cy5.5, the rhodamine derivative JA133, Alexa Fluor 546, Alexa Fluor 633, and Alexa Fluor 647.

11. The method of any one of claims 1 to 8, wherein the donor fluorescent moiety is selected from the group consisting of FITC, phycoerythrin, FAM, Cy3, Cy3.5, R6G, TMR, Alexa Fluor 488, and Alexa Fluor 555.

12. The method of any one of claims 1 to 7, wherein the radioactive moiety is selected from the group consisting of ³²P, ³³P, ³⁵S, ¹²³I, ¹⁸F, ³H, ¹⁴C, and complexes of radioactive metals.

13. The method of any one of claims 1 to 7, wherein the binding moiety is selected from the group consisting of an antigenic peptide, an antigenic small molecule, biotin, and a His-tag.

14. The method of any one of claims 1 to 8, wherein the combination of the donor fluorescent moiety and the acceptor fluorescent moiety capable to fluoresce upon transfer of energy from the donor fluorescent moiety is selected from the group consisting of:
(a) FITC and TRITC;
(b) phycoerythrin and Cy7;
(c) FAM and TMR; and
(d) Alexa Fluor 488 and Alexa Fluor 546.

15. The method of any one of claims 1 to 14, wherein probe 2 molecules to which the first reporter group of probe 1 has been transferred are detected by the fluorescence signal of the first reporter group, by the quenching effect of the first reporter group, by the fluorescence signal of the first reporter group of probe 2, by binding of an optionally labelled antibody, by the radioactive signal, and/or by the binding of streptavidin.

16. The method of any one of claims 1 to 15, wherein a reporter group is transferred from the probe 1 to probe 2 and/or to probe 3 by a chemical reaction selected from the group consisting of
(a) substitution at the carbonyl carbon atom as depicted in reaction scheme (I): 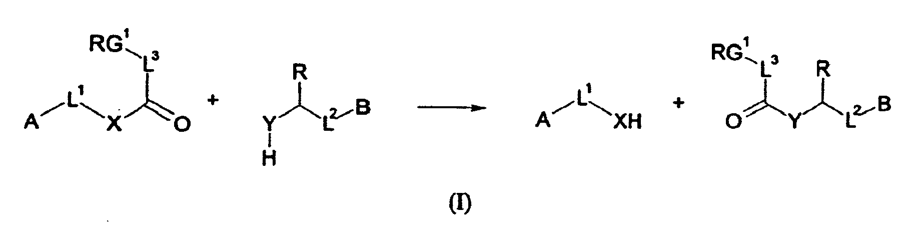 wherein
RG¹ is a first reporter group;
X is S, O, Se, S-C(O), O-C(O), Se-C(O), or P⁺R¹R², wherein the C(O) group, if present, is bound to L¹;
Y is NH, S, N-R⁴, HN-O, NR⁴-NR⁵, O, O-O, O-NH, S-S, S-O, PR³, P(OR³), Se, or a C nucleophile, wherein the S-O group is oriented in such that the O is bound to the carbon atom carrying the R group;
R is hydrogen, an alkyl, alkenyl, alkynyl, cycloalkyl, heterocycloalkyl, aryl, heteroaryl, aralkyl, or a heteroaralkyl group, optionally substituted;
R¹, R², and R³, if present, are independently selected from the group consisting of aryl and alkyl;
R⁴ and R⁵, if present, are independently from each other hydrogen, an alkyl, alkenyl-, alkynyl, cycloalkyl, heterocycloalkyl, aryl, heteroaryl, aralkyl, or a heteroaralkyl group, optionally substituted;
L¹ is a linker or a bond; L² is a linker or a bond; L³ is a linker or a bond;
A comprises a region, which is complementary to a first region of the target nucleic acid sequence, and optionally at least a second reporter group which is linked to said region via a covalent bond or a linker; and
B comprises a region, which is complementary to a second region of the target nucleic acid sequence, and optionally at least a first reporter group which is linked to said region via a covalent bond or a linker;
(b) substitution at the alkyl carbon atom as depicted in reaction scheme (II): 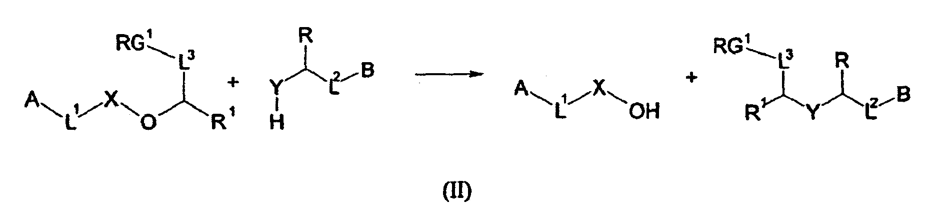 wherein
RG¹ is a reporter group;
X is SO₂ or P⁺R²R³;
Y is NH, S, S-PO₃, N-R⁵, HN-O, NR⁵-NR⁶, O, O-O, ONH, S-S, S-O, PR⁴, P(OR⁴), Se, Se-PO₃, or a C nucleophile, wherein S-PO₃, Se-PO₃, S-O are oriented in such that the -PO₃ moiety or the O atom is bonded to the carbon atom carrying the R residue and S and Se are bonded to H before the reaction and to the carbon atom linked to L³ after the reaction;
R is hydrogen, an alkyl, alkenyl, alkynyl, cycloalkyl, heterocycloalkyl, aryl, heteroaryl, aralkyl, or a heteroaralkyl group, optionally substituted;
R¹ is -CN, -NO₂, -COOAlk, -H, -CHO, -C0Alk;
R², R³, and R⁴ if present, are independently from each other aryl and alkyl;
R⁵ and R⁶, if present, are independently from each other hydrogen, an alkyl, alkenyl, alkynyl, cycloalkyl, heterocycloalkyl, aryl, heteroaryl, aralkyl, or a heteroaralkyl group, optionally substituted;
L¹ is a linker or a bond; L² is a linker or a bond; L³ is a linker or a bond;
A comprises a region, which is complementary to a first region of the target nucleic acid sequence, and optionally a second reporter group which is linked to said region via a covalent bond or a linker; and
B comprises a region, which is complementary to a second region of the target nucleic acid sequence, and optionally a first reporter group which is linked to said region via a covalent bond or a linker;
(c) substitution at phosphate as depicted in reaction scheme (III): 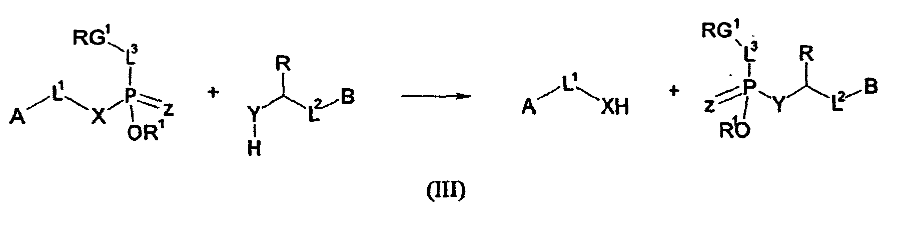 wherein RG¹ is a reporter group;
X is O, NR², or S;
Y is O, NH, Se or S;
Z is not present or O;
R and R¹ are independently from each other hydrogen, an alkyl, alkenyl, alkynyl, cycloalkyl, heterocycloalkyl, aryl, heteroaryl, aralkyl, or a heteroaralkyl group, optionally substituted;
R², if present, is hydrogen, an alkyl, alkenyl, alkynyl, cycloalkyl, heterocycloalkyl, aryl, heteroaryl, aralkyl, or a heteroaralkyl group, optionally substituted;
L¹ is a linker or a bond; L² is a linker or a bond; L³ is a linker or a bond;
A comprises a region, which is complementary to a first region of the target nucleic acid sequence, and optionally a second reporter group which is linked to said region via a covalent bond or a linker; and
B comprises a region, which is complementary to a second region of the target nucleic acid sequence, and optionally a first reporter group which is linked to said region via a covalent bond or a linker;
(d) Staudinger reaction as depicted in reaction scheme (IV): 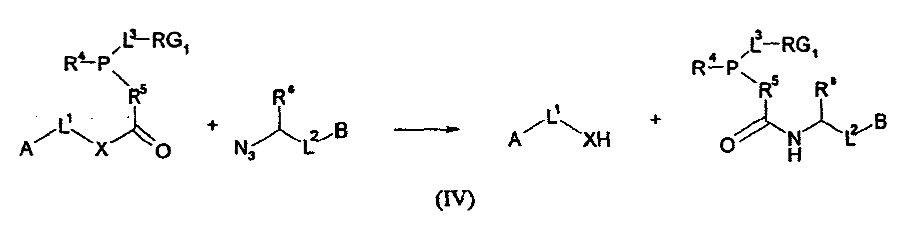 wherein RG₁ is a reporter group;
X is O, S, Se, or NR³, wherein R³ is H or alkyl;
R⁴ is hydrogen, an alkyl, alkenyl, alkynyl, cycloalkyl, heterocycloalkyl, aryl, heteroaryl, aralkyl, or a heteroaralkyl group, optionally substituted;
R⁵ is an alkyl, alkenyl, alkynyl, cycloalkyl, heterocycloalkyl, aryl, heteroaryl, aralkyl, or a heteroaralkyl group, optionally substituted;
R⁶ is hydrogen, an alkyl, alkenyl, alkynyl, cycloalkyl, heterocycloalkyl, aryl, heteroaryl, aralkyl, or a heteroaralkyl group, optionally substituted;
L¹ is a linker or a bond; L² is a linker or a bond; L³ is a linker or a bond;
A comprises a region, which is complementary to a first region of the target nucleic acid sequence, and optionally a second reporter group which is linked to said region via a covalent bond or a linker; and
B comprises a region, which is complementary to a second region of the target nucleic acid sequence, and optionally a first reporter group which is linked to said region via a covalent bond or a linker;
(e) Wittig reaction as depicted in reaction scheme (V): 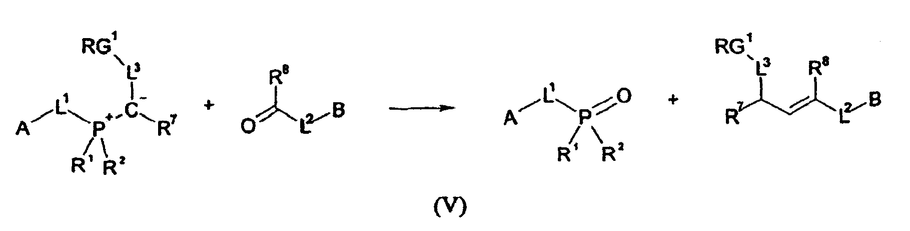
wherein RG₁ is a reporter group;
R¹ and R² are independently from each other selected from the group consisting of hydrogen,
an alkyl, alkenyl, alkynyl, cycloalkyl, heterocycloalkyl, heteroaryl, aralkyl, and a heteroaralkyl group, optionally substituted;
R¹ is C(O)N-alkyl, NO₂, CN, C(O)-alkyl, C(O)O-alkyl, aryl, heteroaryl, fluorinated alkyl;
R⁸ is hydrogen, CH=CH₂, aryl, alkyl;
L¹ is a linker or a bond; L² is a linker or a bond; L³ is a linker or a bond;
A comprises a region, which is complementary to a first region of the target nucleic acid sequence, and optionally a second reporter group which is linked to said region via a covalent bond or a linker; and
B comprises a region, which is complementary to a second region of the target nucleic acid sequence, and optionally a first reporter group which is linked to said region via a covalent bond or a linker;

17. The method of any of claims 1 to 16, wherein the one or more linkers are selected from the group consisting of an alkyl, alkenyl, alkynyl, cycloalkyl, heterocycloalkyl, aryl, heteroaryl, aralkyl, and a heteroaralkyl group, optionally substituted.

18. The method of any of claims 1 to 17, wherein the probe 2 is represented by formula (VI) 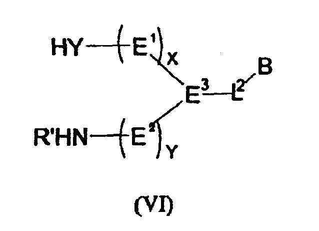 wherein
E₁ and E₂ are independent of each other CHR", wherein R" is hydrogen, alkyl, alkenyl, alkynyl, cycloalkyl, heterocycloalkyl, aryl, heteroaryl, aralkyl, or a heteroaralkyl group;
E³ is selected from the group consisting of alkyl, alkenyl, heteroalkyl, and heteroalkenyl, cycloalkyl, heterocycloalkyl, alicyclic system, aryl or heteroaryl group; optionally substituted; and wherein E¹ and E² are attached to the same or to adjacent carbon and/or nitrogen atom(s);
R' is hydrogen, an alkyl, alkenyl, alkynyl, cycloalkyl, heterocycloalkyl, aryl, heteroaryl, aralkyl, or a heteroaralkyl group;
L² is a linker or a bond;
B comprises a region, which is complementary to a second region of the target nucleic acid sequence, and optionally a first reporter group which is linked to said region via a covalent bond or a linker;
Y is S or Se; and
one of X and Y is 1 and the other one is 0 or both X and Y are 1 or one of X and Y is 2 and the other one is 0;
or formula (VII): 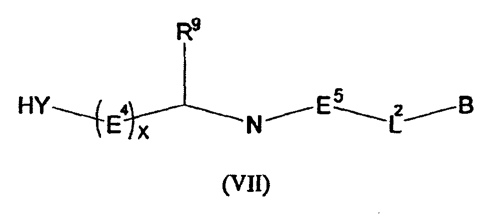 wherein
E⁴ in each instance is independently CHR", wherein R" is hydrogen, an alkyl, alkenyl, alkynyl, cycloalkyl, heterocycloalkyl, aryl, heteroaryl, aralkyl, or a heteroaralkyl group, optionally substituted;
E⁵ is CHR"' or CR"', wherein R"' is hydrogen, an alkyl, alkenyl, alkynyl, cycloalkyl, heterocycloalkyl, aryl, heteroaryl, aralkyl, or a heteroaralkyl group, optionally substituted;
R⁹ is hydrogen; alkyl; alkenyl; alkynyl; cycloalkyl; heterocycloalkyl; aryl; heteroaryl; aralkyl; or a heteroaralkyl group; optionally substituted or R⁹ and R"' are taken together to form a heterocycloalkyl, alicylic system or heteroaryl; optionally substituted;
L² is a linker or a bond;
B comprises a region, which is complementary to a second region of the target nucleic acid sequence, and optionally a first reporter group which is linked to said region via a covalent bond or a linker;
Y is S or Se; and
x is 1 or 2.

19. The method of any one of claims 1 to 18, wherein the distance between the first region and the second region of the target nucleic acid and/or the distance between the first and the third region and/or the distance between the second and the third region ranges from 0 to 10 nucleotides.

20. The method of claims 1 to 19 comprising the additional step of detecting probe 1 and/or probe 3.

21. The method of claims 1 to 20 comprising the following additional steps:
(iv) contacting a probe 1 and a probe 2 in a separate sample, which does not contain the target nucleic acid sequence, and
(v) detecting probe 1 molecules from which said first reporter group has been transferred and/or probe 2 molecules to which said first reporter group has been transferred .

22. The method of any one of claims 1 to 21, wherein probe 2 is immobilized on a stationary phase.

23. The method of claim 22 further comprising a washing step carried out after step (ii) which removes the sample and probe 1.

24. The method of claims 1 to 23, wherein a third probe is added to the sample prior, during or after the transfer of the reporter group from probe 1 to probe 2.

25. The method of any one of claims 1 to 24, wherein the target nucleic acid is DNA or RNA.

26. The method of any one of claims 1 to 25, wherein the target nucleic acid is a prokaryotic, viral or eukaryotic nucleic acid.

27. The method of any one of claims 1 to 26, wherein the target nucleic acid contains a single nucleotide polymorphism (SNP).

28. The method of any one of claims 1 to 27, wherein the target nucleic acid is a splice variant of a naturally occurring nucleic acid.

29. A kit for detecting at least one target nucleic acid sequence in a sample comprising one probe set for each target nucleic acid sequence, the probe set comprising:
(a) a probe 1 having the structure (VIII) 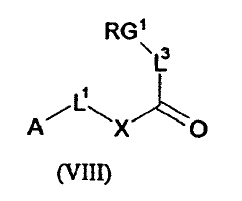 wherein
RG₁ is a first reporter group;
X is S, O, Se, S-C(O), O-C(O), Se-C(O), or P⁺R¹R², wherein the C(O) group, if present, is bound to L¹;
R¹ and R², if present, are independently selected from the group consisting of aryl and alkyl;
L¹ is a linker or a bond; L³ is a linker or a bond;
A comprises a region, which is complementary to a first region of the target nucleic acid sequence, and optionally a second reporter group which is linked to said region via a covalent bond or a linker;
and
(b) a probe 2 having the structure (IX) or (X) 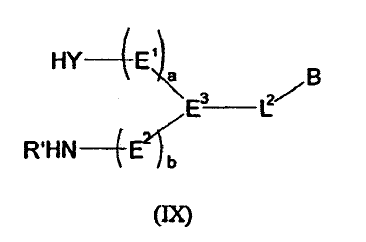
wherein
E¹ and E² are independent of each other CHR", R" being a hydrogen, alkyl, alkenyl, alkynyl, cycloalkyl, heterocycloalkyl, aryl, heteroaryl, aralkyl, or a heteroaralkyl group;
E³ is selected from the group consisting of alkyl, alkenyl, heteroalkyl, and heteroalkenyl, cycloalkyl, heterocycloalkyl, alicyclic system, aryl or heteroaryl group; optionally substituted; and wherein E¹ and E² are attached to the same or to adjacent carbon and/or nitrogen atom(s);
R' is hydrogen, an alkyl, alkenyl, alkynyl, cycloalkyl, heterocycloalkyl, aryl, heteroaryl, aralkyl, or a heteroaralkyl group;
Y is S, or Se;
L² is a linker or a bond;
B comprises a region, which is complementary to a second region of the target nucleic acid sequence, and optionally a first reporter group which is linked to said region via a covalent bond or a linker;
and one of a and b is 1 and the other one is 0 or both a and b are 1 or one of a and b is 2 and the other one is 0;
or 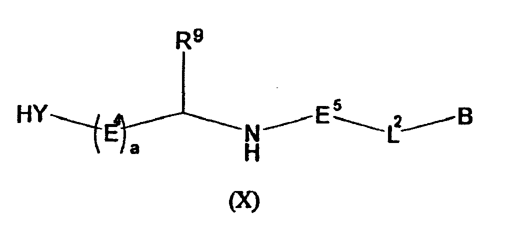 wherein
E⁴ in each instance is independently CHR", wherein R" is hydrogen, an alkyl, alkenyl, alkynyl, cycloalkyl, heterocycloalkyl, aryl, heteroaryl, aralkyl, or a heteroaralkyl group, optionally substituted;
E⁵ is CHR'" or CR"', wherein R"' is hydrogen, an alkyl, alkenyl, alkynyl, cycloalkyl, heterocycloalkyl, aryl, heteroaryl, aralkyl, or a heteroaralkyl group, optionally substituted;
R⁹ is hydrogen; alkyl; alkenyl; alkynyl; cycloalkyl; heterocycloalkyl; aryl; heteroaryl; aralkyl; or a heteroaralkyl group; optionally substituted or R⁹ and R"' are taken together to form a heterocycloalkyl, alicylic system or heteroaryl; optionally substituted;
Y is S, or Se;
L² is a linker or a bond;
B comprises a region, which is complementary to a second region of the target nucleic acid sequence, and optionally a first reporter group which is linked to said region via a covalent bond or a linker; and
a is 1 or 2;
wherein the second region of the target nucleic acid sequence is adjacent to the first region of the target nucleic acid.

30. The kit of claim 29, wherein the regions which are complementary to the first region of the target nucleic acid sequence or to the second region of the target nucleic acid sequence are independently from each other selected from the group consisting of DNA, RNA, PNA, PS-DNA, OMe-RNA, MOE-RNA, NP, FANA, LNA, MF, CeNA and tcDNA.

31. The kit of claim 29 or 30, wherein probe 1 comprises a second reporter group.

32. Use of the method according to any one of claims 1 to 28 or of a kit according to any of claims 29 to 31 for determining the sequence of a target nucleic acid.

33. The use of claim 32 for the detection of at least one single nucleotide polymorphism in at least one target nucleic acid.

34. Use of the method according to any one of claims 1 to 26 or of a kit according to any of claims 29 to 31 for the detection of at least one target nucleic acid from at least one pathogenic organism, preferably a virus, a bacterium, a fungus.

35. Use of the method according to any one of claims 1 to 26 or of a kit according to any of claims 29 to 31 for the detection of at least one target nucleic acid from at least one organism causing allergic reactions.


### Annex to the application documents - subsequently filed sequences listing

SEQUENCE LISTING

<110>  Humboldt-Universität zu Berlin

<120>  Methods for Detecting Target Nucleic Acids Using Template
       Catalyzed Transfer Reactions

<130>  522-3

<140>  EP 06010966
<141>  2006-05-24

<160>  3

<170>  PatentIn version 3.3

<210>  1
<211>  18
<212>  DNA
<213>  Homo sapiens

<400>  1
agaaggggtg tggaggtc                                                    18


<210>  2
<211>  18
<212>  DNA
<213>  Homo sapiens

<400>  2
agaaggggtg tggatgtc                                                    18


<210>  3
<211>  10
<212>  DNA
<213>  Artificial

<220>
<223>  PNA donor probe directed against Ras sequence

<400>  3
caccccttct                                                             10